# EUROPEAN PATENT APPLICATION

(11) **EP 1 801 124 A1**
(43) Date of publication of application: **27.06.2007**
(21) Application number: 05781302.4
(22) Date of filing: 31.08.2005
(51) Int. Cl.: C07K 16/18, A01K 67/027, A61K 38/00, A61K 39/35, A61K 39/395, A61P 15/16, C12N 5/10, C12N 15/09, C12Q 1/02, C12Q 1/68, G01N 33/15, G01N 33/50, C07K 14/47

(54) **UTILIZATION OF SPERM MEMBRANE PROTEIN OBF**

(30) Priority: 31.08.2004 JP 2004253114
(71) Applicant: CHUGAI SEIYAKU KABUSHIKI KAISHA, Tokyo, 115-8543 (JP)
(72) Inventor: OKABE, Masaru, Minoo-shi, Osaka 5620031 (JP); INOUE, Naokazu, Ibaraki-shi, Osaka 5670048 (JP)
(74) Representative: Vossius & Partner
(86) International application number: PCT/JP2005/015847
(87) International publication number: WO 2006/025421

(57) **Abstract**

The present invention yielded unique monoclonal antibodies that reacted specifically only to sperms after acrosome reaction, and the antigens (OBFs) recognized by the antibodies were identified. The antibodies were found to inhibit fusion between mouse sperms and eggs. Furthermore, OBF gene-knockout mice were created and analyzed to clarify the *in vivo* functions of OBF. As a result, male OBF gene-knockout mice were shown to be infertile, and their sperms were shown to be able to bind to eggs, but had no fusion ability.

SEQUENCE LISTING

<110>  CHUGAI SEIYAKU KABUSHIKI KAISHA

<120>  Utilization of sperm membrane protein OBF

<130>  C1-A0411P

<150>  JP 2004-253114
<151>  2004-08-31

<160>  7

<170>  PatentIn version 3.1

<210>  1
<211>  1053
<212>  DNA
<213>  Homo sapiens

<400>  1
atggggccgc attttaccct cctgtgtgcg gcgctggccg gttgcttgct tcctgccgag      60

gggtgtgtta tatgtgaccc gtctgtcgtg ctggcgctaa agtccctgga gaaagattac     120

ctgcctggcc acctggatgc gaagcatcac aaagccatga tggaaagggt agagaatgcc     180

gtgaaggatt tccaggaact gtcgcttaat gaggatgcct atatgggggt cgttgatgag     240

gccacactgc aaaaggggtc ctggagtttg ctgaaggatc tgaaacgcat cacagacagt     300

gatgtaaaag gcgatctctt tgtgaaggag ctattttgga tgttgcactt gcaaaaggaa     360

acctttgcca cctatgttgc tcgattccaa aaagaggctt attgtcccaa caaatgtggt     420

gtgatgttgc aaactctgat ctggtgcaag aactgcaaaa aggaggttca cgcttgtcga     480

aagtcctacg attgcgggga gcggaatgtg gaagttcctc aaatggaaga catgatcctg 540

gactgtgagt taaactggca tcaggcttcg gaaggcctca ctgattacag cttttacagg 600

gtttggggga acaatacgga gaccttggtg tccaagggga aagaggccac cctgaccaag 660

cccatggtgg gtccagagga tgcaggcagc taccgctgcg agctgggctc tgtgaattcc 720

agcccagcca cgatcatcaa ttttcacgtc acagtgttgc ccaaaatgat caaggaggaa 780

aaaccttctc caaatatcgt aaccccgggg gaggcgacca cggagtcgtc cataagcctc 840

cagcctctgc agcccgagaa aatgctggca agccgccttc tggggctgct gatctgcggc 900

tccctagcac tgataaccgg ccttaccttt gcgatatttc gtcgaaggaa ggtgatcgat 960

ttcatcaaat cctcactgtt tggccttggc agtggagttg ccgagcaaac ccaggtccca 1020

aaagaaaagg ccacagattc gaggcaacaa taa 1053

<210> 2
<211> 350
<212> PRT
<213> Homo sapiens

<400> 2

Met Gly Pro His Phe Thr Leu Leu Cys Ala Ala Leu Ala Gly Cys Leu
1               5                   10                  15

Leu Pro Ala Glu Gly Cys Val Ile Cys Asp Pro Ser Val Val Leu Ala
            20                  25                  30

Asp Met Ile Leu Asp Cys Glu Leu Asn Trp His Gln Ala Ser Glu Gly
180 185 190

Leu Thr Asp Tyr Ser Phe Tyr Arg Val Trp Gly Asn Asn Thr Glu Thr
195 200 205

Leu Val Ser Lys Gly Lys Glu Ala Thr Leu Thr Lys Pro Met Val Gly
210 215 220

Pro Glu Asp Ala Gly Ser Tyr Arg Cys Glu Leu Gly Ser Val Asn Ser
225 230 235 240

Ser Pro Ala Thr Ile Ile Asn Phe His Val Thr Val Leu Pro Lys Met
245 250 255

Ile Lys Glu Glu Lys Pro Ser Pro Asn Ile Val Thr Pro Gly Glu Ala
260 265 270

Thr Thr Glu Ser Ser Ile Ser Leu Gln Pro Leu Gln Pro Glu Lys Met
275 280 285

Leu Ala Ser Arg Leu Leu Gly Leu Leu Ile Cys Gly Ser Leu Ala Leu
290 295 300

Ile Thr Gly Leu Thr Phe Ala Ile Phe Arg Arg Arg Lys Val Ile Asp
305 310 315 320

Phe Ile Lys Ser Ser Leu Phe Gly Leu Gly Ser Gly Val Ala Glu Gln
                325                 330                 335

Thr Gln Val Pro Lys Glu Lys Ala Thr Asp Ser Arg Gln Gln
            340                 345                 350

<210>  3
<211>  1194
<212>  DNA
<213>  Mus musculus

<400>  3
atggggccgc attttacact cttgctggca gctcttgcca actgcctgtg tccagggagg      60

ccctgcatca aatgtgacca gtttgtgaca gatgcgctaa agactttcga aaacacttac     120

ctgaatgacc acctgccaca cgacattcac aaaaatgtaa tgaggatggt gaaccatgaa     180

gtatcgagct tcggcgtagt cacttcggct gaggattcct atttgggggc cgtggacgag     240

aacacactgg aacaagcaac ctggagtttt ctgaaggatc tgaagcgtat tacagacagt     300

gacttaaaag gagagctctt tataaaggaa ctattgtgga tgcttcgtca tcaaaaggac     360

atctttaaca atcttgctag acagttccaa aaggaagttc tttgtcccaa caaatgcgga     420

gtgatgtcgc agactttgat ctggtgtctt aagtgcgaaa agcagttgca catttgtcgg     480

aaatccctag attgtggaga gcgccacata gaggtacatc gctcggaaga cctggtcctg     540

gactgtctgc tcagttggca tcgtgcttct aagggactta cagattacag tttttacagg     600

gtttgggaga acagttctga gaccttgatt gccaagggga aagaaccata tctgaccaag     660

tcgatggtgg gtccagagga tgctggcaac taccgctgtg tgctagatac catcaaccaa 720

ggtcatgcca ccgtcatccg ctacgatgtc acagtattgc ccccaaagca ttcagaggaa 780

aaccaaccac cgaacatcat aacccaagag gagcacgaga ctcctgtcca cgtgactcca 840

cagacaccac cggggcagga gccagagtcg gagctgtacc cggagctgca cccagagttg 900

tacccggagc tcatacctac agtcgctcaa aatccagaga aaaaaatgaa aactcgcctc 960

ttaatactgt tgacccttgg ctttgtggtt cttgtggcca gcatcattat ctcggtactt 1020

cactttagga aagtcagcgc taaattgaag aatgcaagtg acgaagtcaa acccaccgcg 1080

tcaggatcca agtcagatca gagtttgtcc caacagatgg gattgaagaa ggcctctcag 1140

gcagatttta actctgacta ctctggagat aagagcgagg caacagaaaa ctaa 1194

<210> 4
<211> 397
<212> PRT
<213> Mus musculus

<400> 4

Met Gly Pro His Phe Thr Leu Leu Leu Ala Ala Leu Ala Asn Cys Leu
1               5                   10                  15

Cys Pro Gly Arg Pro Cys Ile Lys Cys Asp Gln Phe Val Thr Asp Ala
            20                  25                  30

Leu Lys Thr Phe Glu Asn Thr Tyr Leu Asn Asp His Leu Pro His Asp

        35                  40                  45

Ile His Lys Asn Val Met Arg Met Val Asn His Glu Val Ser Ser Phe
    50                  55                  60

Gly Val Val Thr Ser Ala Glu Asp Ser Tyr Leu Gly Ala Val Asp Glu
65                  70                  75                  80

Asn Thr Leu Glu Gln Ala Thr Trp Ser Phe Leu Lys Asp Leu Lys Arg
                85                  90                  95

Ile Thr Asp Ser Asp Leu Lys Gly Glu Leu Phe Ile Lys Glu Leu Leu
            100                 105                 110

Trp Met Leu Arg His Gln Lys Asp Ile Phe Asn Asn Leu Ala Arg Gln
        115                 120                 125

Phe Gln Lys Glu Val Leu Cys Pro Asn Lys Cys Gly Val Met Ser Gln
    130                 135                 140

Thr Leu Ile Trp Cys Leu Lys Cys Glu Lys Gln Leu His Ile Cys Arg
145                 150                 155                 160

Lys Ser Leu Asp Cys Gly Glu Arg His Ile Glu Val His Arg Ser Glu
                165                 170                 175

Asp Leu Val Leu Asp Cys Leu Leu Ser Trp His Arg Ala Ser Lys Gly

            180                 185                 190

Leu Thr Asp Tyr Ser Phe Tyr Arg Val Trp Glu Asn Ser Ser Glu Thr
        195                 200                 205

Leu Ile Ala Lys Gly Lys Glu Pro Tyr Leu Thr Lys Ser Met Val Gly
    210                 215                 220

Pro Glu Asp Ala Gly Asn Tyr Arg Cys Val Leu Asp Thr Ile Asn Gln
225                 230                 235                 240

Gly His Ala Thr Val Ile Arg Tyr Asp Val Thr Val Leu Pro Pro Lys
                245                 250                 255

His Ser Glu Glu Asn Gln Pro Pro Asn Ile Ile Thr Gln Glu Glu His
            260                 265                 270

Glu Thr Pro Val His Val Thr Pro Gln Thr Pro Pro Gly Gln Glu Pro
        275                 280                 285

Glu Ser Glu Leu Tyr Pro Glu Leu His Pro Glu Leu Tyr Pro Glu Leu
    290                 295                 300

Ile Pro Thr Val Ala Gln Asn Pro Glu Lys Lys Met Lys Thr Arg Leu
305                 310                 315                 320

Leu Ile Leu Leu Thr Leu Gly Phe Val Val Leu Val Ala Ser Ile Ile

                325                 330                 335

Ile Ser Val Leu His Phe Arg Lys Val Ser Ala Lys Leu Lys Asn Ala
            340                 345                 350

Ser Asp Glu Val Lys Pro Thr Ala Ser Gly Ser Lys Ser Asp Gln Ser
        355                 360                 365

Leu Ser Gln Gln Met Gly Leu Lys Lys Ala Ser Gln Ala Asp Phe Asn
    370                 375                 380

Ser Asp Tyr Ser Gly Asp Lys Ser Glu Ala Thr Glu Asn
385                 390                 395

<210> 5
<211> 1152
<212> DNA
<213> Rattus norvegicus

<400> 5
atggggctac attttacact cttgctggca gctcttgcca actgcctgtg tccagcaagg 60

ctctgcatca tatgtgaccc gtttgtggtg gctgcaataa agactttgga gcagaattac 120

ctgcctaccc acctggcgcc cgagcatcac gaagatgtaa tgaagagggt agagcaggaa 180

gtgaggaact tcgctgatct gcccttgaat cagaatacct ttctgggggt tgtagatgag 240

gacacactgg aacaagcatc ctggagtttt ctgaaggatc tgaaacgtat cacggacagt 300

gacgtaaaag gagagctctt tgtaaaggag ctattttgga tgcttcgttt gcaaaaggac 360

atctttgcca cccttgttgc acgtttccaa aaggaagttt attgtcctaa ccaatgtggc 420

acgatgtcgc agactctgat ctggtgtaat aagtgtgaga agcagatgca cttctgtcgg 480

aaatccatgg attgtggaga gcgccagata gaggtgcatc gcttggaaga catggtcttg 540

gactgccagc tcagttggca tcacgcttct gagggactta cggattacag tttctacagg 600

gtttggggga acagttctga gaccttgatg tccaagggaa aagaacctta cctgaccaag 660

acgatggtgg gtccagagga tgctggcaac taccgctgtg agctggacac cgtcaacgct 720

ggtcccgcca ccatcatcta ctaccatgtc acagtattgc ccccaaggtc tgtagaggaa 780

aaaccaccgc cgaacatcgt aacccaggag gaggaagaga cccctgtcca ggtgattgta 840

ccgacgctgg agccagagcc ggagccagag cccataccta cagttaccca tcgtccagaa 900

aaaaaattga aaagccgcct cttaatactg ttgatccttg gcttcgtggt tcttgtggcc 960

agcgtcattg cctcggtact tcactttaga aaaaccagag ttaaatcgaa gaactcaaat 1020

gtggaaaaca aaacctctgc ggcagaattc aagtcagagg cggaatcacc ccaaaagatg 1080

ggatcgagga agctctctca ggcagagttt cacactgact cttcggataa ggttgaggaa 1140

gcagataact aa 1152

<210> 6
<211> 383
<212> PRT
<213> Rattus norvegicus

<400> 6

Met Gly Leu His Phe Thr Leu Leu Leu Ala Ala Leu Ala Asn Cys Leu
1               5                   10                  15

Cys Pro Ala Arg Leu Cys Ile Ile Cys Asp Pro Phe Val Val Ala Ala
            20                  25                  30

Ile Lys Thr Leu Glu Gln Asn Tyr Leu Pro Thr His Leu Ala Pro Glu
        35                  40                  45

His His Glu Asp Val Met Lys Arg Val Glu Gln Glu Val Arg Asn Phe
    50                  55                  60

Ala Asp Leu Pro Leu Asn Gln Asn Thr Phe Leu Gly Val Val Asp Glu
65                  70                  75                  80

Asp Thr Leu Glu Gln Ala Ser Trp Ser Phe Leu Lys Asp Leu Lys Arg
                85                  90                  95

Ile Thr Asp Ser Asp Val Lys Gly Glu Leu Phe Val Lys Glu Leu Phe
            100                 105                 110

Trp Met Leu Arg Leu Gln Lys Asp Ile Phe Ala Thr Leu Val Ala Arg
        115                 120                 125

Phe Gln Lys Glu Val Tyr Cys Pro Asn Gln Cys Gly Thr Met Ser Gln
    130                 135                 140

Thr Leu Ile Trp Cys Asn Lys Cys Glu Lys Gln Met His Phe Cys Arg
145                 150                 155                 160

Lys Ser Met Asp Cys Gly Glu Arg Gln Ile Glu Val His Arg Leu Glu
                165                 170                 175

Asp Met Val Leu Asp Cys Gln Leu Ser Trp His His Ala Ser Glu Gly
            180                 185                 190

Leu Thr Asp Tyr Ser Phe Tyr Arg Val Trp Gly Asn Ser Ser Glu Thr
        195                 200                 205

Leu Met Ser Lys Gly Lys Glu Pro Tyr Leu Thr Lys Thr Met Val Gly
    210                 215                 220

Pro Glu Asp Ala Gly Asn Tyr Arg Cys Glu Leu Asp Thr Val Asn Ala
225                 230                 235                 240

Gly Pro Ala Thr Ile Ile Tyr Tyr His Val Thr Val Leu Pro Pro Arg
                245                 250                 255

Ser Val Glu Glu Lys Pro Pro Pro Asn Ile Val Thr Gln Glu Glu Glu
            260                 265                 270

Glu Thr Pro Val Gln Val Ile Val Pro Thr Leu Glu Pro Glu Pro Glu
        275                 280                 285

Pro Glu Pro Ile Pro Thr Val Thr His Arg Pro Glu Lys Lys Leu Lys
    290                 295                 300

Ser Arg Leu Leu Ile Leu Leu Ile Leu Gly Phe Val Val Leu Val Ala
305                 310                 315                 320

Ser Val Ile Ala Ser Val Leu His Phe Arg Lys Thr Arg Val Lys Ser
                325                 330                 335

Lys Asn Ser Asn Val Glu Asn Lys Thr Ser Ala Ala Glu Phe Lys Ser
            340                 345                 350

Glu Ala Glu Ser Pro Gln Lys Met Gly Ser Arg Lys Leu Ser Gln Ala
        355                 360                 365

Glu Phe His Thr Asp Ser Ser Asp Lys Val Glu Glu Ala Asp Asn
    370                 375                 380

<210>  7
<211>  1479
<212>  DNA
<213>  Mus musculus

<220>
<221>  CDS
<222>  (266)..(1459)

<400>  7

gggatgaccg gtgactcttg gagtcagtag aaaaacccta aattactctc cccagtcttc 60

caaggaaaac cttactctta gtcgtgagaa agtattttaa aaagtcctca gaacctggac 120

tgagctgggt gaagcattag cgcttgaggg cgcggaattg gaaagcggct tttaaagggt 180

gttgtttaag cgccctcccc cctgccctct gtttggggtg agaggatcct ctggcggtgc 240

tggcaagctg tgcgttctgg ccttc atg ggg ccg cat ttt aca ctc ttg ctg 292
Met Gly Pro His Phe Thr Leu Leu Leu
1               5

gca gct ctt gcc aac tgc ctg tgt cca ggg agg ccc tgc atc aaa tgt 340
Ala Ala Leu Ala Asn Cys Leu Cys Pro Gly Arg Pro Cys Ile Lys Cys
10                  15                  20                  25

gac cag ttt gtg aca gat gcg cta aag act ttc gaa aac act tac ctg 388
Asp Gln Phe Val Thr Asp Ala Leu Lys Thr Phe Glu Asn Thr Tyr Leu
                30                  35                  40

aat gac cac ctg cca cac gac att cac aaa aat gta atg agg atg gtg 436
Asn Asp His Leu Pro His Asp Ile His Lys Asn Val Met Arg Met Val
            45                  50                  55

aac cat gaa gta tcg agc ttc ggc gta gtc act tcg gct gag gat tcc 484
Asn His Glu Val Ser Ser Phe Gly Val Val Thr Ser Ala Glu Asp Ser
        60                  65                  70

tat ttg ggg gcc gtg gac gag aac aca ctg gaa caa gca acc tgg agt 532
Tyr Leu Gly Ala Val Asp Glu Asn Thr Leu Glu Gln Ala Thr Trp Ser
    75                  80                  85

ttt ctg aag gat ctg aag cgt att aca gac agt gac tta aaa gga gag 580
Phe Leu Lys Asp Leu Lys Arg Ile Thr Asp Ser Asp Leu Lys Gly Glu
90                  95                  100                 105

ctc ttt ata aag gaa cta ttg tgg atg ctt cgt cat caa aag gac atc 628
Leu Phe Ile Lys Glu Leu Leu Trp Met Leu Arg His Gln Lys Asp Ile
                110                 115                 120

ttt aac aat ctt gct aga cag ttc caa aag gaa gtt ctt tgt ccc aac 676
Phe Asn Asn Leu Ala Arg Gln Phe Gln Lys Glu Val Leu Cys Pro Asn
            125                 130                 135

aaa tgc gga gtg atg tcg cag act ttg atc tgg tgt ctt aag tgc gaa 724
Lys Cys Gly Val Met Ser Gln Thr Leu Ile Trp Cys Leu Lys Cys Glu
        140                 145                 150

aag cag ttg cac att tgt cgg aaa tcc cta gat tgt gga gag cgc cac 772
Lys Gln Leu His Ile Cys Arg Lys Ser Leu Asp Cys Gly Glu Arg His
    155                 160                 165

ata gag gta cat cgc tcg gaa gac ctg gtc ctg gac tgt ctg ctc agt 820
Ile Glu Val His Arg Ser Glu Asp Leu Val Leu Asp Cys Leu Leu Ser
170                 175                 180                 185

tgg cat cgt gct tct aag gga ctt aca gat tac agt ttt tac agg gtt 868
Trp His Arg Ala Ser Lys Gly Leu Thr Asp Tyr Ser Phe Tyr Arg Val
                190                 195                 200

tgg gag aac agt tct gag acc ttg att gcc aag ggg aaa gaa cca tat 916
Trp Glu Asn Ser Ser Glu Thr Leu Ile Ala Lys Gly Lys Glu Pro Tyr
            205                 210                 215

ctg acc aag tcg atg gtg ggt cca gag gat gct ggc aac tac cgc tgt 964
Leu Thr Lys Ser Met Val Gly Pro Glu Asp Ala Gly Asn Tyr Arg Cys
        220                 225                 230

gtg cta gat acc atc aac caa ggt cat gcc acc gtc atc cgc tac gat 1012
Val Leu Asp Thr Ile Asn Gln Gly His Ala Thr Val Ile Arg Tyr Asp
    235                 240                 245

gtc aca gta ttg ccc cca aag cat tca gag gaa aac caa cca ccg aac     1060
Val Thr Val Leu Pro Pro Lys His Ser Glu Glu Asn Gln Pro Pro Asn
250                 255                 260                 265

atc ata acc caa gag gag cac gag act cct gtc cac gtg act cca cag     1108
Ile Ile Thr Gln Glu Glu His Glu Thr Pro Val His Val Thr Pro Gln
                270                 275                 280

aca cca ccg ggg cag gag cca gag tcg gag ctg tac ccg gag ctg cac     1156
Thr Pro Pro Gly Gln Glu Pro Glu Ser Glu Leu Tyr Pro Glu Leu His
            285                 290                 295

cca gag ttg tac ccg gag ctc ata cct aca gtc gct caa aat cca gag     1204
Pro Glu Leu Tyr Pro Glu Leu Ile Pro Thr Val Ala Gln Asn Pro Glu
        300                 305                 310

aaa aaa atg aaa act cgc ctc tta ata ctg ttg acc ctt ggc ttt gtg     1252
Lys Lys Met Lys Thr Arg Leu Leu Ile Leu Leu Thr Leu Gly Phe Val
    315                 320                 325

gtt ctt gtg gcc agc atc att atc tcg gta ctt cac ttt agg aaa gtc     1300
Val Leu Val Ala Ser Ile Ile Ile Ser Val Leu His Phe Arg Lys Val
330                 335                 340                 345

agc gct aaa ttg aag aat gca agt gac gaa gtc aaa ccc acc gcg tca     1348
Ser Ala Lys Leu Lys Asn Ala Ser Asp Glu Val Lys Pro Thr Ala Ser
                350                 355                 360

gga tcc aag tca gat cag agt ttg tcc caa cag atg gga ttg aag aag     1396
Gly Ser Lys Ser Asp Gln Ser Leu Ser Gln Gln Met Gly Leu Lys Lys
            365                 370                 375

gcc tct cag gca gat ttt aac tct gac tac tct gga gat aag agc gag     1444
Ala Ser Gln Ala Asp Phe Asn Ser Asp Tyr Ser Gly Asp Lys Ser Glu
        380                 385                 390

gca aca gaa aac taa taaagatttg tatttgactt 1479
Ala Thr Glu Asn
    395

## Description

### Technical Field

The present invention relates to uses of the sperm membrane protein OBF (oocyte binding/fusion factor) and of antibodies against OBF.

### Background Art

Fertilization is the beginning of life and thus a very important phenomenon, yet its molecular biological mechanism is unknown. In recent years, however, the creation of genetically altered animals using gene targeting methods has led to reports of factors important in fertilization. However, with regards to the fusion step, only CD9 on eggs has recently been reported as an essential molecule for fusion (Non-Patent Document 1). By identifying sperm's fusion factors, not only is basic research on fertilization advanced, but clinical applications are also expected since they may be applied to contraceptive vaccines that use their antigens.

To date the present inventors have reported the following regarding the sperm membrane protein OBF (oocyte binding/fusion factor): first, a number of monoclonal antibodies (Mab) were produced using sperms as antigens. Of these, the antibody named "OBF13" had a sperm staining pattern that changed during culture, and this condition was revealed to correlate with sperm fertility (Non-Patent Document 2). OBF13 inhibited fertilization (fusion) in *in vitro* fertilization systems; however, it did not inhibit fusion with hamster (Non-Patent Document 3). Furthermore, the inventors have reported that fertilization (fusion) is inhibited by passive immunity in which ascites fluids are administered to females (Non-Patent Document 4). In addition, the inventors have also reported that sperms reactive to OBF 13 can be divided into three types of staining pattern and can be separated using FACS (Non-Patent Document 5).
[Non-Patent Document 1] Miyado, K. et al., Science (2000) 287, 321-324.
[Non-Patent Document 2] Capacitation related changes in antigen distribution on mouse sperm heads and its relation to fertilization rate in vitro. J. Reproductive Immunology, 11, 91-100 (1987).
[Non-Patent Document 3] Effect of monoclonal anti-mouse sperm antibody (OBF13) on the interaction of mouse sperm with zona-free mouse and hamster eggs. J. Reproductive Immunology 13, 211-219 (1988).
[Non-Patent Document 4] An anti-mouse sperm monoclonal antibody (OBF13) and its inhibitory activity on fertilization. Journal of the Japanese Society of Fertility and Sterility, 33, 473-478 (1988).
[Non-Patent Document 5] OBF13 J. Reproductive. Immunology. 16, 71-82 (1989).

### Disclosure of the Invention

### Problems to be Solved by the Invention

The present invention was achieved in view of the above circumstances. An objective of the present invention is to determine fusion factors on the sperm, to provide these factors and antibodies against these factors, and to provide uses thereof. More specifically, an objective is to provide antibodies against the factors, contraceptives comprising these antibodies, contraceptive vaccines that use these factors, uses of OBF proteins or fragments thereof as factors involved in the binding or fusion between sperms and eggs, nonhuman animals in which the functions of genes encoding these factors have been suppressed, methods of screening for contraceptives using these factors, and methods and agents for testing infertility that use these factors as indicators.

### Means to Solve the Problems

The present inventors conducted dedicated studies to achieve the objectives described above. First, the inventors obtained unique monoclonal antibodies that react specifically to sperms only after acrosome reaction and identified the antigen (OBF) recognized by these antibodies. When treated with this antibody, mouse sperms had a normal capacity to bind to and penetrate egg zona pellucida; however, the capacity of the subsequent fusion with the eggs was inhibited by the antibody treatment in a concentration-dependent manner. In addition, OBF gene-knockout mice were generated and analyzed to elucidate the *in vivo* functions of OBF. The result showed that OBF gene-knockout male mice were infertile and although the sperms of OBF-knockout mice could bind to mouse eggs, they had no ability to fuse with them. Experiments using naked hamster eggs capable of fusing with the sperms of various animal species revealed that the sperms of OBF-knockout mice could bind to naked hamster eggs, but could not fuse with them at all. Further, experiments using human OBF polyclonal antibodies revealed that human sperms could not even bind to naked hamster eggs, let alone fuse with them. These results reveal that OBF is an essential molecule for the binding or fusion with eggs during fertilization, regardless of animal species. Fertility could be recovered in OBF-deficient mice by expressing OBF as a transgene. Therefore, there is a possibility that infertility having such cause can be treated by adding an OBF gene or protein. The protein referred to as "OBF" herein is called "Izumo" in other documents and the like (for example, Nature 434, 234-238 (2005)).

The present invention provides the following (1)-(17):
(1) an antibody (other than an OBF13 antibody) with the activity of inhibiting binding or fusion between a sperm and an egg, whose antigen is the protein described in any one of (a) to (d) below, or a fragment thereof:
   (a) a protein encoded by a DNA comprising the nucleotide sequence of any one of SEQ ID NOs: 1, 3, and 5;
   (b) a protein comprising the amino acid sequence of any one of SEQ ID NOs: 2, 4, and 6;
   (c) a protein comprising an amino acid sequence with a substitution, deletion, insertion, and/or addition of one or more amino acids in the amino acid sequence of any one of SEQ ID NOs: 2, 4, and 6, which is functionally equivalent to a protein comprising the amino acid sequence of any one of SEQ ID NOs: 2, 4, and 6; and
   (d) a protein encoded by a naturally derived DNA that hybridizes under stringent conditions to a DNA comprising the nucleotide sequence of any one of SEQ ID NOs: 1, 3, and 5, which is functionally equivalent to a protein comprising the amino acid sequence of any one of SEQ ID NOs: 2, 4, and 6;
(2) a contraceptive comprising as an active ingredient an antibody with the activity of inhibiting binding or fusion between a sperm and an egg, whose antigen is the protein of any one of (a) to (d) below, or a fragment thereof:
   (a) a protein encoded by a DNA comprising the nucleotide sequence of any one of SEQ ID NOs: 1, 3, and 5;
   (b) a protein comprising the amino acid sequence of any one of SEQ ID NOs: 2, 4, and 6;
   (c) a protein comprising an amino acid sequence with a substitution, deletion, insertion, and/or addition of one or more amino acids in the amino acid sequence of any one of SEQ ID NOs: 2, 4, and 6, which is functionally equivalent to a protein comprising the amino acid sequence of any one of SEQ ID NOs: 2, 4, and 6; and
   (d) a protein encoded by a naturally derived DNA that hybridizes under stringent conditions to a DNA comprising the nucleotide sequence of any one of SEQ ID NOs: 1, 3, and 5, which is functionally equivalent to a protein comprising the amino acid sequence of any one of SEQ ID NOs: 2, 4, and 6;
(3) a contraceptive vaccine comprising as an active ingredient the protein of (2) or a fragment thereof;
(4) a use of the protein of (2),or a fragment thereof, as a factor involved in binding or fusion between a sperm and an egg;
(5) a method of screening for a candidate compound for a contraceptive, which comprises the steps of:
   (a) contacting a test compound with the protein of (2) or a fragment thereof;
   (b) detecting binding between the test compound and the protein of (2) or a fragment thereof; and
   (c) selecting a test compound that binds to the protein of (2) or a fragment thereof;
(6) a method of screening for a candidate compound for a contraceptive, which comprises the steps of:
   (a) contacting a test compound with a cell comprising a DNA encoding the protein of (2);
   (b) measuring the expression level of the DNA encoding the protein of (2) in the cell; and
   (c) selecting a compound that reduces the expression level of the DNA as compared to when the test compound is not contacted;
(7) a method of screening for a candidate compound for a contraceptive, which comprises the steps of:
   (a) administering a test compound to a nonhuman animal comprising a DNA encoding the protein of (2);
   (b) measuring the expression level of the DNA encoding the protein of (2) in the nonhuman animal; and
   (c) selecting a compound that reduces the expression level of the DNA as compared to when the test compound is not administered;
(8) a method of screening for a candidate compound for a contraceptive, which comprises the steps of:
   (a) providing a cell or a cell extract comprising a DNA in which a reporter gene is operatively linked downstream of a promoter region of a gene encoding the protein of (2);
   (b) contacting a test compound with the cell or cell extract;
   (c) measuring the expression level of the reporter gene in the cell or cell extract; and
   (d) selecting a compound that reduces the expression level of the reporter gene as compared to when the test compound is not contacted;
(9) a method of screening for a candidate compound for a contraceptive, which comprises the steps of:
   (a) contacting a sperm of a male animal with a compound selected by using a combination of one or more of the methods of (5) to (8);
   (b) confirming the fertility of the sperm; and
   (c) selecting a compound that inhibits the fertility of the sperm;
(10) a method of screening for a candidate compound for a contraceptive, which comprises the steps of:
   (a) administering a nonhuman male animal with a compound selected by using a combination of one or more of the methods of (5) to (8);
   (b) confirming the fertility of a sperm of the nonhuman male animal; and
   (c) selecting a compound that inhibits the fertility of the sperm of the nonhuman male animal;
(11) a nonhuman animal in which a function of a gene encoding the protein of (2) is artificially suppressed;
(12) a nonhuman animal cell in which a function of a gene encoding the protein of (2) is artificially suppressed;
(13) a method of screening for a candidate compound for treating infertility, which comprises the steps of:
   (a) administering a test compound to the nonhuman animal of (11);
   (b) assessing whether the test compound substitutes for a function of the protein of (2); and
   (c) selecting a compound that substitutes for a function of the protein as compared to when the test compound is not administered;
(14) a method of testing for infertility, which comprises the step of measuring the expression level of a gene encoding the protein of (2);
(15) a method of testing for infertility, which comprises the step of detecting a mutation in a region of a gene encoding the protein of (2);
(16) a test agent used in the method of (14) or (15), which comprises an oligonucleotide with a length of at least 15 nucleotides that hybridizes to a region of a gene encoding the protein of (2); and
(17) a test agent used in the method of (14), which comprises an antibody whose antigen is the protein of (2) or a fragment thereof.

### Brief Description of the Drawings

Fig. 1(A) is a photograph of fluorescence immunostaining of sperms using OBF13 antibody before and after acrosome reaction. From the top: 1, fresh sperms; 2, sperms whose membrane permeability was enhanced by freeze-thawing; 3, sperms in which acrosome reaction was induced during culture; 4, sperms in which acrosome reaction was induced during culture. (B) is a photograph of fluorescence immunostaining using OBF13 antibody after penetration through egg zona pellucida.
Fig. 2 is photographs and a graph showing the result of a fertilization inhibition experiment using naked eggs allowed to take up Hoechst 33342.
Fig. 3(A) is photographs showing the result of Western blotting of mouse sperms using two-dimensional electrophoresis and OBF13 monoclonal antibody.
Fig. 3(B) shows the primary structure of the identified OBF13 antigen. In this diagram, the signal sequence is underlined; the immunoglobulin-like domain is boxed; and the transmembrane region is indicated with a dashed underline. A disulfide bond is expected to form between the cysteines at residues 182 and 233.
Fig. 3(C) is a photograph showing a Western blotting of each tissue using a polyclonal antibody against mouse OBF.
Fig. 3(D) schematically shows the primary structure of OBF protein.
Fig. 4(A) shows the structure of a targeting vector in which the OBF gene is replaced with the neomycin resistance gene. (B) to (D) are photographs showing the result of evaluating mice for the presence or absence of homologous recombination using Southern blotting, Northern blotting, and Western blotting.
Fig. 5(A) is a graph of the average number of littermates born to male and female OBF-knockout mice. (B) is a graph showing the result of *in vitro* fertilization (IVF) using OBF-knockout sperms. (C) is a graph showing the results of analyzing fusion between OBF-knockout sperms and eggs. (D) is photographs showing the results of analyzing *in vitro* fertilization and fusion using OBF-knockout sperms and CD9-knockout eggs.
Fig. 6(A) is photographs showing the results of analyzing fusion between mice OBF -/sperms and naked hamster eggs. (B) is photographs and a graph showing the results of analyzing fusion between human sperms and naked hamster eggs using the human OBF polyclonal antibody.
Fig. 7 is photographs showing that fertility is recovered when the cDNA sequence of the OBF gene is integrated into OBF-knockout mice.

### Best Mode for Carrying Out the Invention

The present invention provides antibodies (other than OBF13 antibodies) with the activity of inhibiting binding or fusion between sperms and eggs, whose antigen is a sperm membrane protein OBF, which is essential for binding or fusion between sperms and eggs, or fragments thereof.

The present invention also provides contraceptives comprising as an active ingredient an antibody (comprising OBF13 antibodies) whose antigen is an OBF or fragment thereof, or contraceptive vaccines comprising as an active ingredient an OBF or fragment thereof.

The OBF antigens of the present invention include proteins encoded by DNAs comprising the nucleotide sequence of any one of SEQ ID NOs: 1, 3, and 5, or fragments thereof; proteins comprising the amino acid sequence of any one of SEQ ID NOs: 2, 4, and 6, or fragments thereof; proteins comprising an amino acid sequence with a substitution, deletion, insertion, and/or addition of one or more amino acids in the amino acid sequence of any one of SEQ ID NOs: 2, 4, and 6, and which are functionally equivalent to a protein comprising the amino acid sequence of any one of SEQ ID NOs: 2, 4, and 6, and fragments thereof; and proteins encoded by naturally derived DNAs that hybridize under stringent conditions to a DNA comprising the nucleotide sequence of any one of SEQ ID NOs: 1, 3, and 5, and which are functionally equivalent to a protein comprising the amino acid sequence of any one of SEQ ID NOs: 2, 4, and 6, and fragments thereof.

The above "proteins comprising the amino acid sequence of any one of SEQ ID NOs: 2, 4, and 6" include proteins comprising the amino acid sequence of any one of SEQ ID NOs: 2, 4, and 6, and fusion proteins comprising a protein comprising the amino acid sequence of any one of SEQ ID NOs: 2, 4, and 6.

Meanwhile, the above "proteins comprising an amino acid sequence with a substitution, deletion, insertion, and/or addition of one or more amino acids in the amino acid sequence of any one of SEQ ID NOs: 2, 4, and 6" include proteins comprising an amino acid sequence with a substitution, deletion, insertion, and/or addition of one or more amino acids in the amino acid sequence of any one of SEQ ID NOs: 2, 4, and 6, and fusion proteins comprising such proteins. Such substitutions, deletions, insertions, and/or additions occur naturally. The number of amino acids substituted, deleted, inserted, and/or added is typically 30 amino acids or less, preferably 15 amino acids or less, more preferably five amino acids or less (for example, three amino acids or less), and still more preferably two amino acids or less.

The other proteins or peptides to be fused with the proteins of the present invention are not particularly limited, as long as the fusion proteins are functionally equivalent to the proteins of the present invention. The other peptides to be fused with the proteins of the present invention include known peptides such as, for example, FLAG (Hopp, T.P. et al., Biotechnology (1988) 6, 1204-1210), 6x His which comprises six His (histidine) residues, and 10x His. Examples of other proteins to be fused to the proteins of the present invention include GST (glutathione-S-transferase) and such.

Herein, "functionally equivalent" means that a protein of interest has the same biological or biochemical function as that of a protein comprising the amino acid sequence of any one of SEQ ID NOs: 2, 4, and 6. Such biological functions include functions essential for binding or fusion between sperms and eggs. Whether a protein of interest has the same features as a protein comprising the amino acid sequence of any one of SEQ ID NOs: 2, 4, and 6 can be assessed, for example, by obtaining an antibody against the protein of interest and testing whether the antibody inhibits binding or fusion between sperms and eggs.

Proteins functionally equivalent to a protein comprising the amino acid sequence of any one of SEQ ID NOs: 2, 4, and 6 have a common or similar structural feature to that of a protein comprising the amino acid sequence of any one of SEQ ID NOs: 2, 4, and 6. Such structural features include, for example, the presence of a signal sequence, an extracellular region, a transmembrane region, and a cytoplasmic region; the presence of a single immunoglobulin-like (Ig-like) domain in the extracellular region; and the presence of an N-linked glycosylation sequence in the Ig-like domain.

Those skilled in the art can appropriately select hybridization conditions for isolating DNAs encoding proteins functionally equivalent to an OBF protein. Conditions for hybridization may include, for example, those with low stringency. Examples of conditions of low stringency include post-hybridization washing in 5x SSC and 0.1% SDS at 42°C, and preferably in 5x SSC and 0.1% SDS at 50°C. More preferable hybridization conditions include those of high stringency. Highly stringent conditions include, for example, washing in 0.1x SSC and 0.1% SDS at 65°C. In these conditions, the higher the temperature, the more it can be expected that a DNA with a high homology would be obtained. However, several factors such as temperature and salt concentration can influence hybridization stringency, and those skilled in the art can suitably select these factors to accomplish similar stringencies.

DNAs encoding proteins functionally equivalent to OBF protein can also be isolated using gene amplification methods, for example, a polymerase chain reaction (PCR) using primers synthesized based on the sequence information of a DNA encoding an OBF protein (SEQ ID NO: 1,3, or 5).

These proteins functionally equivalent to an OBF protein and that are encoded by DNAs isolated by hybridization techniques or gene amplification techniques generally have high homology to an OBF protein in their amino acid sequences. High homology typically refers to at least 50% or higher identity, preferably 75% or higher identity, more preferably 85% or higher identity, and still more preferably 95% or higher identity to the amino acid sequence of any one of SEQ ID NOs: 2, 4, and 6. Amino acid or nucleotide sequence identity can be determined using the BLAST algorithm of Karlin and Altschul (Proc. Natl. Acad. Sci. USA 90:5873-5877, 1993). Programs called BLASTN and BLASTX were developed based on this algorithm (Altschul et al., J. Mol. Biol. 215:403-410, 1990). When nucleotide sequences are analyzed by BLASTN based on BLAST, the parameter settings are, for example, as follows: score = 100 and wordlength = 12. When amino acid sequences are analyzed by BLASTX based on BLAST, the parameter settings are, for example, as follows: score = 50 and wordlength = 3. When BLAST and Gapped BLAST programs are used, default parameters are used in each program. Specific procedures for the above analytical methods are known (http://www.ncbi.nlm.nih.gov.).

The antigen fragments of the present invention include, for example, Ig-like domains (for example, amino acids 167-253 of mouse OBF), but are not limited thereto.

The antibodies of the present invention are not particularly limited, as long as they can recognize an OBF protein and inhibit binding or fusion between sperms and eggs. The antibodies may be polyclonal or monoclonal antibodies. Moreover, mouse antibodies, rat antibodies, rabbit antibodies, sheep antibodies, camel antibodies, chimeric antibodies, humanized antibodies, human antibodies, and such can be appropriately used as the antibodies of the present invention. Furthermore, low molecular-sized antibodies and such can also be used as the antibodies of the present invention.

Polyclonal and monoclonal antibodies can be generated by methods known to those skilled in the art. Hybridomas producing monoclonal antibodies can be generated basically using known techniques by the following procedure: an antigen of interest or cells expressing the antigen of interest are used as sensitizing antigens; immunization is carried out using these and following standard immunization methods; the resulting immune cells are fused with known parental cells using standard cell fusion methods; and monoclonal cells that produce antibodies (hybridomas) are screened using standard screening methods. For example, mammals such as mice, rats, rabbits, sheep, and monkeys can be used as the immunized animals. Antigens can be prepared by known methods, for example, methods using baculovirus (WO 98/46777). When OBF expressed on the membrane of baculovirus is used as the immunogen, gp64 transgenic mice (Japanese Patent Application No. 2002-180351; Japanese Patent Application No. 2002-164834) can be used as animals to be immunized.

Hybridomas can be generated, for example, according to the method of Milstein *et al.* (Kohler, G. and Milstein, C., Methods Enzymol. (1981) 73:3-46). When the antigen has low immunogenicity, immunization can be performed using the antigen bound to immunogenic macromolecules, such as albumin.

Moreover, recombinant antibodies produced using gene recombination techniques, in which antibody genes are cloned from hybridomas and inserted into appropriate vectors and the resulting vectors are introduced into hosts, can be used (see, for example, Carl, A. K. Borrebaeck, James, W. Larrick, Therapeutic Monoclonal Antibodies, Published in the United Kingdom by Macmillan Publishers, 1990). Specifically, cDNAs for antibody variable regions (V regions) are synthesized from the mRNAs of hybridomas using reverse transcriptase. When a DNA encoding an antibody V region of interest is obtained, the DNA is linked with a DNA encoding a desired constant region (C region) of an antibody, and this is incorporated into an expression vector. Alternatively, the DNA encoding the antibody V region may be incorporated into an expression vector comprising the DNA of the antibody C region. The DNA is inserted into an expression vector such that it is expressed under the regulation of the expression regulatory region, for example, an enhancer or promoter. Then, host cells are transformed with this expression vector and the antibodies can be expressed.

In the present invention, artificially modified recombinant antibodies, such as chimeric and humanized antibodies, can be used to reduce heterologous antigenicity against humans. These modified antibodies can be produced using known methods. A chimeric antibody is an antibody comprising the variable regions of the heavy and light chains of an antibody from a nonhuman mammal such as a mouse, and the constant regions of the heavy and light chains of a human antibody. The chimeric antibody can be produced by linking a DNA encoding the variable regions of the mouse antibody with a DNA encoding the constant regions of the human antibody, incorporating this into an expression vector, and then introducing the vector into a host.

Humanized antibodies are also referred to as "reshaped human antibodies". They are antibodies in which the complementarity determining region (CDR) of an antibody derived from a nonhuman mammal, for example a mouse, is grafted to the CDR of a human antibody, and general gene recombination procedures for this are also known. Specifically, a DNA sequence designed to link a murine antibody CDR to the framework region (FR) of a human antibody is synthesized by PCR, using several oligonucleotides produced such that they comprise overlapping portions in the terminal parts. The obtained DNA is linked to a DNA encoding human antibody constant regions, and this is then integrated into an expression vector, and the antibody is produced by introducing this vector into host cells (see European Patent Application EP 239400, and International Patent Application WO 96/02576). The human antibody FR to be linked via the CDR is selected so the CDR forms a favorable antigen-binding site. Amino acids in the framework region of the antibody variable region may be substituted as necessary, such that the CDR of the reshaped human antibody forms a suitable antigen-binding site (Sato, K. et al., 1993, Cancer Res. 53, 851-856).

Methods for obtaining human antibodies are also known. For example, human lymphocytes can be sensitized *in vitro* with a desired antigen, or with cells expressing the desired antigen, and the sensitized lymphocytes can be fused with human myeloma cells such as U266 to obtain the desired human antibody with antigen-binding activity (Japanese Patent Application Kokoku Publication No. (JP-B) H01-59878 (examined, approved Japanese patent application published for opposition)). Further, a desired human antibody can be obtained by using a desired antigen to immunize transgenic animals that have a full repertoire of human antibody genes (see International Patent Application WO 93/12227, WO 92/03918, WO 94/02602, WO 94/25585, WO 96/34096, and WO 96/33735). Furthermore, techniques for obtaining human antibodies by panning using a human antibody library are also known. For example, variable regions of human antibodies can be expressed as single chain antibodies (scFvs) on the surface of phages using phage display methods, and phages that bind to antigens can be selected. The DNA sequences that encode the variable regions of the human antibodies binding the antigens can be determined by analyzing the genes of the selected phages. By determining the DNA sequences of the scFvs that bind to the antigens, appropriate expression vectors carrying the sequences can be produced to yield human antibodies. These methods are known, and the following publications can be referred to: WO 92/01047, WO 92/20791, WO 93/06213, WO 93/11236, WO 93/19172, WO 95/01438, and WO 95/15388.

When the antibody genes are isolated and introduced into appropriate hosts to produce the antibodies, appropriate combinations of hosts and expression vectors can be used. Eukaryotic host cells that can be used are animal cells, plant cells, and fungal cells. Known animal cells include: (1) mammalian cells, for example, CHO, COS, myeloma, baby hamster kidney (BHK), HeLa, and Vero cells; (2) amphibian cells, for example, *Xenopus* oocytes; or (3) insect cells, for example, sf9, sf21, and Tn5. Known plant cells include cells derived from the genus *Nicotiana,* for example *Nicotiana tabacum,* and calluses may be cultured from these cells. Known fungal cells include yeast cells, for example, the genus *Saccharomyces,* such as *Saccharomyces cerevisiae*; and filamentous fungi, for example, the genus *Aspergillus* such as *Aspergillus niger.* When prokaryotic cells are used, bacterial cells can be used in the production systems. Known bacterial cells include *Escherichia coli* and *Bacillus subtilis.* The antibodies can be obtained by inserting the antibody genes of interest into these cells by transformation, and then culturing the transformed cells *in vitro.*

Moreover, the antibodies may be low molecular-sized antibodies as long as they bind to an OBF protein and inhibit a function of an OBF protein. The low molecular-sized antibodies are not particularly limited, as long as they comprise antibody fragments in which a portion is deleted from a whole antibody (for example, whole IgG and such), and they have the ability to bind to an antigen. The antibody fragments of the present invention are not particularly limited, as long as they are portions of whole antibodies; however, they preferably comprise a heavy chain variable region (VH) and/or a light chain variable region (VL). The amino acid sequence of a VH or VL may also comprise substitutions, deletions, additions, and/or insertions. Furthermore, some portions may also be deleted from a VH and/or VL, as long as they have the ability to bind to an antigen. Moreover, variable regions may also be chimeric or humanized. Specific examples of the antibody fragments include Fab, Fab', F(ab')2, and Fv. Further, specific examples of the low molecular-sized antibodies include Fab, Fab', F(ab')2, Fv, scFv (single chain Fv), diabodies, and sc(Fv)2 (single chain (Fv)2).

Herein, an "Fv" fragment is the smallest antibody fragment, and comprises a complete antigen recognition site and a binding site. An "Fv" fragment is a dimer (VH-VL dimer) in which one VH and one VL are strongly linked by a noncovalent bond. The three complementarity determining regions (CDRs) of each of the variable regions interact to form an antigen-binding site on the surface of the VH-VL dimer. Six CDRs form an antigen binding site on an antibody. However, even one variable region (or a half Fv, which comprises only three antigen-specific CDRs) can recognize and bind to an antigen, although its affinity is lower than that of the entire binding site.

scFv comprises the VH and VL regions of an antibody, and these regions exist on a single polypeptide chain. In general, an Fv further comprises a peptide linker between the VH and VL, and an scFv can therefore form a structure necessary for antigen binding (see Pluckthun "The Pharmacology of Monoclonal Antibodies" Vol.113 (Rosenburg and Moore ed. (Springer Verlag, New York) pp. 269-315,1994) for a review on scFv). Linkers in the present invention are not particularly limited, as long as they do not inhibit the expression of the antibody variable regions linked to their ends.

A DNA encoding an scFv can be obtained by amplifying by PCR using, as a template, either an entire DNA, or a partial DNA encoding a desired amino acid sequence, selected from a DNA encoding the H chain or H chain V region of the above antibody and a DNA encoding the L chain or L chain V region of the above antibody; and using a primer pair that defines the two ends. Further amplification can be subsequently conducted using a combination of the DNA encoding the peptide linker portion, and primer pairs that define both ends of that DNA such that they are each ligated to the H chain and L chain. Once DNAs encoding scFvs are constructed, expression vectors comprising these DNAs, and hosts transformed with these expression vectors can be obtained by conventional methods. By using these hosts, scFvs can be obtained according to conventional methods. These antibody fragments can be produced by hosts by obtaining and expressing genes in the same way as outlined above.

"Diabodies" refer to divalent (bivalent) antibody fragments constructed by gene fusion (Holliger P. et al., Proc. Natl. Acad. Sci. USA 90: 6444-6448 (1993); EP 404,097; WO 93/11161). A diabody is a dimer composed of two polypeptide chains; generally, in each of the polypeptide chains a VL and a VH on the same chain are connected via a short linker, for example, a linker of about five residues, so that they cannot bind to each other. Because the linker between the VL and the VH in the same polypeptide chain is short, the two cannot form a single chain variable region fragment, but instead form a dimer. Thus, a diabody comprises two antigen-binding sites.

sc(Fv)2 is a single-chain low molecular-sized antibody, prepared by linking two VH and two VL with linkers and such (Hudson et al., J. Immunol. Methods 1999; 231: 177-189). sc(Fv)2 can be produced, for example, by linking scFv with linkers. The order of the two VH and the two VL is not particularly limited and may be any order.

Linkers such as arbitrary peptide linkers that can be introduced by genetic engineering or synthetic linker compounds (see for example Protein Engineering, 9(3), 299-305, 1996) can be used as the linkers linking the variable regions of an antibody. The length of the polypeptide linkers is not particularly limited and can be suitably selected according to the purpose by those skilled in the art. Normally, the length is 1-100 amino acids, preferably 3-50 amino acids, more preferably 5-30 amino acids, and even more preferably 12-18 amino acids (for example, 15 amino acids). However, the length and sequence of the peptide linkers can be suitably selected according to the purpose by those skilled in the art.

Synthetic linker compounds (chemical crosslinking agents) include, crosslinking agents usually used to crosslink peptides, for example, *N*-hydroxysuccinimide (NHS), disuccinimidyl suberate (DSS), bis(sulfosuccinimidyl)suberate (BS³), dithiobis(succinimidyl propionate) (DSP), dithiobis(sulfosuccinimidyl propionate) (DTSSP), ethylene glycol bis(succinimidyl succinate) (EGS), ethylene glycol bis(sulfosuccinimidyl succinate) (sulfo-EGS), disuccinimidyl tartrate (DST), disulfosuccinimidyl tartrate (sulfo-DST), bis[2-(succinimidooxycarbonyloxy)ethyl]sulfone (BSOCOES), and bis[2-(sulfosuccinimidooxycarbonyloxy)ethyl]sulfone (sulfo-BSOCOES). These crosslinking agents are commercially available. In general, three linkers are required to link four antibody variable regions. The linkers to be used may be of the same or different types.

Modified antibodies for use include antibodies linked to various molecules, such as polyethylene glycols (PEG). Moreover, antibodies can also be linked to radioisotopes, chemotherapeutic agents, cytotoxic substances such as bacteria-derived toxins, etc. Such modified antibodies can be prepared by chemically modifying an obtained antibody. Methods for modifying antibodies are already established in the art.

Antibodies for use in the present invention may also be bispecific antibodies. A bispecific antibody may comprise antigen-binding sites that recognize different epitopes on an OBF protein molecule. Alternatively, one of the antigen-binding sites may recognize an OBF protein, and the other may recognize a radioactive substance, chemotherapeutic agent, or cytotoxic substance such as a cell-derived toxin. The bispecific antibodies can be produced by linking HL pairs from two types of antibodies, or they can be obtained by fusing hybridomas which produce different monoclonal antibodies to produce fused cells producing bispecific antibodies. Further, bispecific antibodies can also be produced using genetic engineering techniques.

Antibodies in which sugar chains have been modified can also be used in the present invention. Techniques for modifying antibody sugar chains are already known (for example, WO 00/61739 and WO 02/31140). An "antibody" of the present invention also includes these antibodies.

The antibodies expressed or produced as described above can be purified by known methods that are used for conventional protein purification. The antibodies can be separated and purified, for example, by appropriately selecting or combining methods such as affinity columns such as protein A columns, column chromatography, filtration, ultrafiltration, salting out, or dialysis (Antibodies A Laboratory Manual, Ed Harlow, David Lane, Cold Spring Harbor Laboratory, 1988).

The antigen-binding activity of antibodies (Antibodies A Laboratory Manual. Ed Harlow, David Lane, Cold Spring Harbor Laboratory, 1988) can be measured using known techniques. For example, ELISA (enzyme linked immunosorbent assay), EIA (enzyme immunoassay), RIA (radioimmunoassay), or fluoroimmunoassay can be used.

Known methods can be used to measure whether a specific molecule binds to an OBF protein. Known methods include, for example, immunoprecipitation, Western blotting, ELISA, EIA, RIA, fluoroimmunoassays, and methods using biosensors that utilize the surface plasmon resonance phenomenon.

The antibodies of the present invention can be used as contraceptives. The subjects to be administered with the antibodies are not particularly limited and include, for example, humans, pets, domestic animals, wild animals, and birds.

The contraceptives of the present invention can be administered both orally and parenterally, but are preferably administered parenterally. Specific examples include injections, nasal administrations, pulmonary administrations, cutaneous administrations, and transvaginal administrations. For example, injections can be administered systemically or locally by intravenous injections, intramuscular injections, intraperitoneal injections, or subcutaneous injections. Moreover, the methods of administration can be appropriately selected according to the age and symptoms of the patients. The dose can be selected, for example, from within the range of 0.0001 mg to 1,000 mg per kilogram of body weight per administration. Alternatively, doses can be selected, for example, from within the range of 0.001-100,000 mg/body for each patient.

When the antibodies of the present invention are used as contraceptives, they can be formulated by methods known to those skilled in the art. Contraceptives comprising antibodies of the present invention, which are used for such therapeutic purposes, can be formulated by mixing as necessary with suitable pharmaceutically acceptable carriers, vehicles, or such that do not react with the antibodies. Such carriers and vehicles include, for example, sterilized water, physiological saline, stabilizers, diluents, antioxidants (ascorbic acid and such), buffers (phosphate, citrate, other organic acids, and such), preservatives, detergents (PEG, Tween, and such), chelating agents (EDTA and such), and binders. Moreover, other low-molecular-weight polypeptides; proteins such as serum albumin, gelatin and immunoglobulins; amino acids such as glycine, glutamine, asparagine, arginine, and lysine; carbohydrates and sugars such as polysaccharides and monosaccharides; and sugar alcohols such as mannitol and sorbitol may be comprised. Aqueous solutions used for injections include, for example, physiological saline and isotonic solutions comprising glucose or other adjunctive agents such as D-sorbitol, D-mannose, D-mannitol, and sodium chloride. They may also be combined with appropriate solubilizing agents, such as alcohols (ethanol, etc), polyalcohols (propylene glycol, PEG, etc), or non-ionic detergents (polysorbate 80 or HCO-50).

The present invention provides contraceptive vaccines comprising as an active ingredient an OBF antigen (an OBF or a fragment thereof). The OBF antigens for the contraceptive vaccines of the present invention can be prepared as natural proteins, and also as recombinant proteins by using known gene recombination techniques. Moreover, the OBF antigens for the contraceptive vaccines of the present invention may be prepared in the state of being expressed in cells. The species from which the OBF antigens for the contraceptive vaccines of the present invention originate are not particularly limited and can be appropriately selected depending on the species that are the subject of administration of the contraceptive vaccines.

The natural proteins can be prepared, for example, from extracts of OBF protein-expressing tissues, such as testes and sperms, by methods that use affinity chromatography using antibodies against OBF protein.

The recombinant proteins can be prepared by methods known to those skilled in the art. For example, they can be prepared as OBF proteins or fragments thereof. OBF proteins or fragments thereof can be prepared, for example, by the following procedure: a DNA encoding an OBF protein is incorporated into an appropriate expression vector; this is introduced into suitable host cells, and the obtained transformants are collected; an extract is obtained and purified by subjecting it to chromatography, such as ion-exchange, reverse, or gel filtration chromatography, or to affinity chromatography which uses a column onto which antibodies against OBF protein have been immobilized, or to a combination of several of these columns.

When an OBF protein is expressed in host cells (for example, animal cells, *Escherichia coli,* and such) as a fusion protein with glutathione S-transferase or as an OBF protein to which multiple histidine residues have been attached, the expressed OBF protein can be purified using a glutathione column or nickel column. A fusion protein can be produced, for example, by linking a DNA encoding an OBF protein in frame with a DNA encoding another protein or peptide, introducing this into an expression vector, then expressing it in a host.

With regards to the above vectors, for example, when the host is *E. coli,* as long as the vector has an "ori" for amplification in *E. coli,* such that vectors are amplified and prepared in large quantities in *E. coli* (for example, JM109, DH5α, HB101, and XLlBlue) or such, and further has a selection gene for transformed *E. coli* (for example, a drug resistance gene that allows discrimination using a certain drug (ampicillin, tetracycline, kanamycin, or chloramphenicol)), the vectors are not particularly limited. The vectors include, for example, M13 vectors, pUC vectors, pBR322, pBluescript, and pCR-Script. In addition to the above vectors, for example, pGEM-T, pDIRECT, and pT7 can also be used for the subcloning and excision of cDNAs. When using vectors to produce the OBF proteins of this invention, expression vectors are particularly useful. When an expression vector is expressed in *E. coli,* for example, it should have the above characteristics in order to be amplified in *E. coli.* Additionally, when *E. coli* such as JM109, DH5 α, HB101, or XL1-Blue are used as the host, the vector must have a promoter that allows efficient expression in *E. coli,* for example, a lacZ promoter (Ward et al. (1989) Nature 341:544-546; (1992) FASEB J. 6:2422-2427), araB promoter (Better et al. (1988) Science 240:1041-1043), or T7 promoter. Other examples of the vectors include pGEX-5X-1 (Pharmacia), "QIAexpress system" (QIAGEN), pEGFP, and pET.

Furthermore, the vector may comprise a signal sequence for protein secretion. When producing proteins into the periplasm of *E. coli,* the pelB signal sequence ( Lei, S. P. et al. J. Bacteriol. 169:4379 (1987)) may be used as a signal sequence for protein secretion. For example, calcium chloride methods or electroporation methods may be used to introduce the vector into a host cell.

In addition to *E. coli,* expression vectors derived from mammals (e.g., pcDNA3 (Invitrogen), pEGF-BOS (Nucleic Acids Res. (1990) 18(17):5322), pEF, pCDM8), insect cells (e.g., "Bac-to-BAC baculovirus expression system" (GIBCO-BRL), pBacPAK8), plants (e.g., pMHl, pMH2), animal viruses (e.g., pHSV, pMV, pAdexLcw), retroviruses (e.g., pZIPneo), yeasts (e.g., "Pichia Expression Kit" (Invitrogen), pNV11, SP-Q01), and *Bacillus subtilis* (e.g., pPL608, pKTH50) may also be used as vectors for producing the OBF proteins.

In order to express proteins in animal cells, such as CHO, COS, and NIH3T3 cells, the vector must have a promoter necessary for expression in such cells, for example, an SV40 promoter (Mulligan et al. (1979) Nature 277:108), MMLV-LTR promoter, EF1αpromoter (Mizushima et al. (1990) Nucleic Acids Res. 18:5322), CMV promoter, etc.). It is even more preferable that the vector comprises a gene for selecting transformants (for example, a drug-resistance gene enabling discrimination by a drug (such as neomycin and G418)). Examples of vectors with such characteristics include pMAM, pDR2, pBK-RSV, pBK-CMV, pOPRSV, pOP13, and such.

Also, production systems using animals or plants may be used as systems for producing proteins *in vivo.* A DNA encoding an OBF protein may be introduced into an animal or plant, and the OBF protein produced in the body of the animal or plant is then recovered.

When using animals, there are production systems which use mammals or insects. Mammals such as goats, pigs, sheep, mice, and cattle may be used (Vicki Glaser SPECTRUM Biotechnology Applications (1993)). Alternatively, the mammals may be transgenic animals.

For example, a DNA encoding an OBF protein may be prepared as a fusion gene with a gene encoding a protein specifically produced in milk, such as the goat β-casein gene. DNA fragments comprising this fusion gene are injected into goat embryos, which are then transplanted to female goats. The OBF protein can then be obtained from milk produced by the transgenic goats, which are born from the goats that received the embryos, or from their offspring. Appropriate hormones may be administered to the transgenic goats to increase the volume of milk comprising the protein produced by the transgenic goats (Ebert, K.M. et al., Bio/Technology 12, 699-702 (1994)).

Insects, such as silkworms, may also be used. Baculoviruses into which a DNA encoding an OBF protein has been inserted can be used to infect silkworms, and the OBF protein can be obtained from their body fluids (Susumu, M. et al., Nature 315, 592-594 (1985)).

When using plants, tobacco can be used, for example. When tobacco is used, a DNA encoding an OBF protein is inserted into a plant expression vector, for example, pMON 530, and the vector is introduced into a bacterium such as *Agrobacterium tumefaciens.* These bacteria are then used to infect tobacco, such as *Nicotiana tabacum,* and the OBF proteins can be obtained from the leaves of the tobacco (Julian K.-C. Ma et al., Eur. J. Immunol. 24, 131-138 (1994)).

The resulting OBF proteins can be isolated from the inside or outside (such as the medium) of host cells, and purified as substantially pure and homogenous proteins. Any standard method for separating and purifying proteins may be used, and methods are not limited to any specific method. Proteins can be separated and purified by appropriately selecting or combining, for example, chromatographic columns, filtration, ultrafiltration, salting out, solvent precipitation, solvent extraction, distillation, immunoprecipitation, SDS-polyacrylamide gel electrophoresis, isoelectric focusing, dialysis, recrystallization, and such.

The OBF proteins of the present invention can be arbitrarily modified, or can have peptides partially removed from them, by reacting appropriate protein-modifying enzymes with the proteins before or after purification. Such protein-modifying enzymes include, for example, trypsin, chymotrypsin, lysyl endopeptidase, protein kinases, and glucosidases.

Pharmaceutically acceptable carriers may be added to the vaccine preparations of the present invention. Herein, "pharmaceutically acceptable carriers" refer to pharmaceutically acceptable materials that are substances different from the antigen (or immunogen) and that can be administered with the antigen during vaccination. Examples include vaccine additives such as adjuvants, preservatives, and stabilizers, but are not limited thereto. Without limitation, adjuvants such as aluminum phosphate, aluminum hydroxide, and MF59 may be used as approved adjuvants for use in human vaccine.

Without limitation, about 0.2% gelatin or dextran, 0.1-1.0% sodium glutamate, approximately 5% lactose, approximately 2% sorbitol, or such may be used as a stabilizer. Without limitation, about 0.01% thimerosal, about 0.1% beta-propionolactone, about 0.5% phenoxyethanol or such may be used as a stabilizer.

Injections are prepared by adding pH-control agents, buffers, stabilizers, preservatives or such if necessary and made into subcutaneous, intramuscular, and intravenous injections by common procedures. An injection can be prepared as a solid formulation before use by, for example, freeze-drying a solution after storing it in a container. A single dose can be stored in a container, or doses may be stored in a same container.

Various known methods can be used for administering the vaccines of the present invention. Examples of administration include subcutaneous injection, intramuscular injection, intranasal administration, oral administration, percutaneous administration, and transvaginal administration.

A suitable vaccination method is determined by considering the type of vaccine antigen, dosage form, timing of antibody expression, duration of antibody persistence, age of the subject receiving vaccine, and such. The method of vaccination is ultimately determined through clinical testing by specialists, and these methods are known to those skilled in the art. The product may be for single inoculation or for multiple inoculations. The dosage changes depending on the weight and age of the patient, the method of administration and such, but those skilled in the art can appropriately select a suitable dose.

The OBF proteins of the present invention or fragments thereof can be used as factors involved in binding or fusion between sperms and eggs.

Examples of uses as factors involved in binding or fusion between sperms and eggs include the following, but are not limited thereto. For example, in an *in vitro* fertilization system, fertility can be recovered by adding an OBF protein or a fragment thereof to the sperms of infertile men whose infertility is caused by OBF deficiency or dysfunction. Conversely, contraceptive effects can be induced by competitively inhibiting the binding between OBF proteins on sperms and OBF protein-binding molecules on eggs by directly administering OBF proteins or fragments thereof into the vagina.

The present inventors discovered that fertilization is inhibited by inhibiting OBF proteins using antibodies, and by suppressing the expression of OBF proteins. This result indicates that screening for contraceptives can be carried out using OBF proteins as targets.

The test compounds to be used in the screening methods of the present invention are not particularly limited and include, for example, single compounds such as natural compounds, organic compounds, inorganic compounds, proteins, antibodies, and peptides; and compound libraries, expression products of gene libraries, antibody libraries, cell extracts, cell culture supernatants, products of fermenting microorganisms, extracts of marine organisms, and plant extracts.

The first embodiment of the screening methods of the present invention relates to screening for compounds that bind to OBF proteins or fragments thereof. In this screening, first, an OBF protein or a fragment thereof is contacted with test compounds. Then, binding between the OBF protein or fragment thereof and test compounds is detected. Next, test compounds that bind to the OBF protein or fragment thereof are selected. Compounds isolated by this method can be candidate compounds for contraceptives. They can also be used as test compounds in the screening methods described below.

There are many methods known to those skilled in the art that can be used to screen for proteins that bind to OBF proteins, using OBF proteins. Such screening can be carried out, for example, by immunoprecipitation. Specifically, the procedure is as follows: a DNA encoding an OBF protein is inserted into an expression vector for foreign genes, such as pSV2neo, pcDNA I, or pCD8, to express the gene in animal cells or the like. Any commonly used promoters may be used for the expression; such promoters include SV40 early promoter (Rigby in Williamson (ed.), Genetic Engineering, Vol. 3. Academic Press, London, p.83-141(1982)), EF-la promoter (Kim et al., Gene 91, p.217-223 (1990)), CAG promoter (Niwa et al. Gene 108, p.193-200 (1991)), RSV LTR promoter (Cullen Methods in Enzymology 152, p.684-704 (1987), SRa promoter (Takebe et al. Mol. Cell. Biol. 8, p.466 (1988)), CMV immediate early promoter (Seed and Aruffo Proc. Natl. Acad. Sci. USA 84, p. 3365-3369 (1987)), SV40 late promoter (Gheysen and Fiers J. Mol. Appl. Genet. 1, p. 385-394 (1982)), Adenovirus late promoter (Kaufman et al. Mol. Cell. Biol. 9, p. 946 (1989)), and HSV TK promoter.

Foreign genes can be introduced and expressed in animal cells by any methods, including electroporation (Chu, G. et al. Nucl. Acid Res. 15, 1311-1326 (1987)), calcium phosphate methods (Chen, C and Okayama, H. Mol. Cell. Biol. 7, 2745-2752 (1987)), DEAE-dextran methods (Lopata, M. A. et al. Nucl. Acids Res. 12, 5707-5717 (1984); Sussman, D. J. and Milman, G. Mol. Cell. Biol. 4, 1642-1643 (1985)), and methods using lipofectin (Derijard, B. Cell 7, 1025-1037 (1994); Lamb, B. T. et al. Nature Genetics 5, 22-30 (1993); Rabindran, S. K. et al. Science 259, 230-234 (1993)).

When a recognition site (epitope) for a monoclonal antibody with known specificity is introduced into an OBF protein at its N- or C-terminus, the OBF protein can be expressed as a fusion protein comprising the recognition site for the monoclonal antibody. Commercially available epitope-antibody systems can be used (Experimental Medicine, 13, 85-90 (1995)). Vectors that allow expression of a fusion protein with β-galactosidase, maltose-binding protein, glutathione S-transferase, green fluorescence protein (GFP), or such via the multiple cloning site are commercially available. Furthermore, there are reports describing methods for preparing fusion proteins in which only small epitope portions comprising several to dozens of amino acids are introduced, such that the properties of the OBF proteins are altered as little as possible when they are made into fusion proteins. For example, epitopes, such as poly-histidine (His-tag), influenza hemagglutinin (HA), human c-myc, FLAG, vesicular stomatitis virus glycoprotein (VSV-GP), T7 gene 10 protein (T7-tag), human herpes simplex virus glycoprotein (HSV-tag), E-tag (epitopes on monoclonal phages), and monoclonal antibodies that recognize the epitopes can be used as epitope-antibody systems to screen for proteins that bind to OBF proteins (Experimental Medicine, 13, 85-90 (1995)).

In immunoprecipitation, these antibodies are added to cell lysates prepared using appropriate detergents to form immune complexes. Such immune complexes comprise OBF proteins, proteins with binding ability thereto, and antibodies. Besides using the antibodies against the above-described epitopes, immunoprecipitation can also be carried out using antibodies against OBF proteins. Antibodies against OBF proteins can be prepared, for example, by introducing a DNA encoding an OBF protein into an appropriate *E. coli* expression vector, expressing this in *E. coli;* purifying the expressed protein, then using this to immunize rabbits, mice, rats, goats, chickens, or such. Alternatively, they can be prepared by immunizing the above-described animals with a synthetic partial peptide of an OBF protein.

When, for example, the antibody is a mouse IgG, the immune complexes can be precipitated using Protein A Sepharose or Protein G Sepharose. Alternatively, when OBF protein is prepared, for example, as a fusion protein with an epitope such as GST, immune complexes can be formed, as is the case when using antibodies against OBF proteins, by using substances that bind specifically to the epitope, such as glutathione Sepharose 4B.

Immunoprecipitation can be carried out, for example, by using or by referring to the standard methods described in the literature (Harlow, E. and Lane, D., "Antibodies", pp. 511-552, Cold Spring Harbor Laboratory publications, New York (1988)).

SDS-PAGE is generally used to analyze immunoprecipitated proteins; the bound proteins can be analyzed using their molecular weights by using gels with suitable concentration. In such cases, it is generally difficult to detect proteins bound to OBF proteins by using standard protein staining methods, such as Coomassie staining and silver staining; thus, detection sensitivity can be improved by culturing cells in culture medium containing the radioisotopes ³⁵S-methionine or ³⁵S-cysteine, to label proteins within the cells, and detecting the radioisotopes. Once the molecular weight of a protein has been determined, a target protein can be purified directly from SDS-polyacrylamide gels, and its sequence can also be determined.

Meanwhile, proteins that bind to OBF proteins or fragments thereof can be isolated, for example, using the method of Skolnik *et al.* (Skolnik, E. Y et al., Cell (1991) 65, 83-90). Specifically, phage vectors (λgt11, ZAP, and the like) are used to generate cDNA libraries from cells or tissues expressing proteins predicted to bind with OBF proteins or fragments thereof. The libraries are expressed on LB-agarose, and the expressed proteins or fragments thereof are immobilized on filters. The above filters are reacted with purified and labeled OBF proteins or fragments thereof, and plaques expressing proteins that bound to OBF proteins or fragments thereof can be detected using the labels. Methods for labeling OBF proteins or fragments thereof include methods using the binding between biotin and avidin; methods using antibodies that specifically bind to OBF proteins or fragments thereof, or to proteins fused with OBF proteins or fragments thereof (for example, GST); methods using radioisotopes; and methods using fluorescence.

Moreover, the first embodiment of the screening methods of the present invention includes methods that use the two-hybrid system using cells (Fields, S., and Sternglanz, R., Trends. Genet. (1994) 10, 286-292; Dalton S, and Treisman R (1992) Characterization of SAP-1, a protein recruited by serum response factor to the c-fos serum response element. Cell 68, 597-612; "Matchmarker Two-Hybrid System", "Mammalian Matchmarker Two-Hybrid Assay Kit", "Matchmarker One-Hybrid System" (all from Clontech), "HybriZAP Two-Hybrid Vector System" (Stratagene)).

The following procedure is used in a two-hybrid system: an OBF protein or fragment thereof (partial peptide) is fused with a DNA-binding region of SRF or a DNA-binding region of GAL4, and is expressed in yeast cells; a library of cDNAs designed to express fusion proteins with a VP16 or GAL4 transcription activation region is prepared from cells that are predicted to express proteins that bind to an OBF protein or fragment thereof; this is introduced into the above yeast cells; and cDNAs derived from the library are isolated from the positive clones detected (when a protein that binds to an OBF protein is expressed in yeast cells, the reporter gene is activated by the binding of these two, and positive clones can be confirmed). Proteins encoded by these cDNAs can be obtained by introducing and expressing the isolated cDNAs in *E. coli.* Thus, proteins that bind to OBF proteins, or their genes, can be prepared by this procedure.

Reporter genes used in the two-hybrid system include, for example, the HIS3 gene, Ade2 gene, LacZ gene, CAT gene, luciferase gene, and PAI-1 (Plasminogen activator inhibitor type 1) gene, but are not limited thereto. Screening based on the two-hybrid method can be carried out using, other than yeast, mammalian cells or such.

Compounds that bind to OBF proteins or fragments thereof can also be screened using affinity chromatography. For example, OBF proteins or fragments thereof are immobilized onto carriers on affinity columns, and test compounds predicted to express proteins that bind to OBF proteins or fragments thereof are applied thereto. Such test compounds include, for example, cell extracts and cell lysates. After the test compounds have been applied, the column is washed and proteins that have bound to OBF proteins or fragments thereof can be prepared.

DNAs encoding the proteins can be obtained by analyzing the amino acid sequences of the obtained proteins, synthesizing oligo DNAs based on these sequences, and screening cDNA libraries using these DNAs as probes.

Examples of methods used to isolate not just proteins but compounds that bind to OBF proteins or fragments thereof, where these methods are known to those skilled in the art, include methods in which synthetic compounds, natural product banks, or random phage peptide display libraries are reacted with immobilized OBF proteins or fragments thereof and molecules that bind to the OBF proteins or fragments thereof are screened, and screening methods that use high-throughput based on combinatorial chemistry technology (Wrighton NC; Farrell FX; Chang R; Kashyap AK; Barbone FP; Mulcahy LS; Johnson DL; Barrett RW; Jolliffe LK; Dower WJ., Small peptides as potent mimetics of the protein hormone erythropoietin, Science (United States) Jul. 26, 1996, 273, p. 458-64; Verdine GL., The combinatorial chemistry of nature. Nature (England) Nov. 7, 1996, 384 p. 11-13; Hogan JC Jr., Directed combinatorial chemistry. Nature (England) Nov. 7, 1996, 384 p. 17-9).

In the present invention, biosensors using the surface plasmon resonance phenomenon can also be used as means for measuring or detecting bound compounds. Biosensors that use the surface plasmon resonance phenomenon enable observation of interactions between test compounds and OBF proteins or fragments thereof in real time as surface plasmon resonance signals, using minute amounts of proteins and without labeling (for example, BIAcore; Pharmacia).

In an alternative embodiment of the screening methods of the present invention, compounds that reduce the expression level of DNAs encoding OBF proteins are screened. Compounds that reduce the expression level of DNAs encoding OBF proteins can be candidate compounds for contraceptives.

In such screenings, the test compounds are first contacted with cells comprising DNAs encoding OBF proteins. Examples of cells (including human cells) that comprise DNAs encoding OBF proteins include sperms, spermatids and muscle cells. Such cells include sperms and spermatids in mice, and sperms, spermatids, and muscle cells in humans. Moreover, in the present invention, "contact" can be carried out, for example, by adding test compounds to cell culture media. In this screening, the expression level in the cells of the DNAs encoding OBF proteins is then determined, and compounds that reduce the DNA expression level compared to without contact with the test compound are selected.

The DNA expression levels can be measured by methods known to those skilled in the art. For example, DNA expression levels can be measured by extracting mRNAs according to conventional methods, and carrying out Northern hybridization or RT-PCR using these mRNAs as templates. Further, DNA expression levels can also be measured using DNA array technology. The translation level of genes can also be measured by collecting fractions comprising OBF proteins following conventional methods and detecting the expression of the OBF proteins by electrophoresis such as SDS-PAGE. Moreover, the translation level of genes can also be measured by performing Western blotting, dot blotting, immunoprecipitation, enzyme-linked immunosorbent assay (ELISA), immunofluorescence, and such using antibodies against OBF proteins to detect the expression of OBF proteins.

In an alternative embodiment of the screening for compounds that reduce the expression level of DNAs encoding OBF proteins, first, test compounds are administered to nonhuman animals comprising a DNA encoding an OBF protein. Nonhuman animals comprising a DNA encoding an OBF protein which can be used include, for example, experimental animals such as mice, rats, rabbits, guinea pigs, dogs, pigs, cats, monkeys, gerbils, and hamsters, but are not limited thereto.

Test compounds can be administered to nonhuman animals orally or parenterally, for example, but the administration is not limited thereto. When the test compound is a protein, for example, a viral vector comprising the gene encoding the protein can also be constructed, and the gene can be introduced into nonhuman animals using the infectivity of the viral vector.

In this screening, the expression level of the DNAs encoding the OBF proteins in the nonhuman animals is then measured, and compounds that reduce the DNA expression level compared to when the test compound is not administered are selected. DNA expression levels can be measured by the methods described above.

Compounds that reduce OBF gene expression levels can also be screened using reporter gene systems. First, cells or cell extracts that comprise a DNA in which a reporter gene is operatively linked downstream of an OBF gene promoter region are provided. Herein, "operatively linked" means that the reporter gene is linked with the OBF gene promoter region such that reporter gene expression is induced when transcription factors bind to the OBF gene promoter region. Therefore, even cases where a reporter gene is linked to another gene and forms a fusion protein with the other gene product are included in the above meaning of "operatively linked", as long as fusion protein expression is induced upon the binding of transcription factors to the OBF gene promoter region.

The above-described reporter genes are not particularly limited, as long as their expression is detectable; for example, the reporter genes include the CAT gene, lacZ gene, luciferase gene, β-glucuronidase gene (GUS), and GFP gene, which are generally used by those skilled in the art.

In this screening, test compounds are then contacted with the cells or cell extracts described above. Then, the expression level of the above reporter genes in the cells or cell extracts is measured. The expression level of a reporter gene can be measured by methods known to those skilled in the art, depending on the type of the reporter gene used. For example, when the reporter gene is a CAT gene, the expression level of the reporter gene can be measured by detecting the acetylation of chloramphenicol by the gene product. When the reporter gene is a lacZ gene, the expression level of the reporter gene can be measured by detecting the color development of a chromogenic compound due to the catalytic effect of the gene expression product. When the reporter gene is a luciferase gene, the expression level of the reporter gene can be measured by detecting the fluorescence of a fluorogenic compound due to the catalytic effect of the gene expression product. When the reporter gene is a β-glucuronidase gene (GUS), the expression level of the reporter gene can be measured by detecting the luminescence of Glucuron (ICN) or color development of 5-bromo-4-chloro-3-indolyl-β-glucuronide (X-Gluc) due to the catalytic effect of the gene expression product. When the reporter gene is a GFP gene, the expression level of the reporter gene can be measured by detecting the fluorescence of GFP protein.

In this screening, compounds that reduce the expression level of the reporter gene as compared to when no test compounds are contacted are selected next.

In an alternative embodiment of the screening methods of the present invention, compounds selected by using a combination of one or more of the screening methods described above or below are first contacted with sperms collected from male animals including humans. Then, sperm fertility is confirmed and compounds that inhibit their fertility are selected. Fertility can be confirmed, for example, using as an indicator whether fertilization occurs when the sperms are contacted with unfertilized eggs collected from females of the same animal species, whether the sperms fuse with naked hamster eggs, or such. The collection of animal sperms and unfertilized eggs, contact of sperms with compounds, contact of sperms with unfertilized eggs, and confirmation of fertilization can be carried out using methods known to those skilled in the art. Moreover, for example, the post-fertilization swelling of sperm heads can be observed, the subsequent pronucleus formation can be observed using a phase contrast microscope, or culture can be continued to observe the egg cleavage.

In still another embodiment of the screening methods of the present invention, first, compounds selected by a combination of one or more of the screening methods described above are administered into male nonhuman animals. Then, the fertility of the sperms of male nonhuman animals is confirmed, and compounds that inhibit fertility are selected. Fertility can be confirmed, for example, using the following as indicators: whether females of a nonhuman animal species have litters when mated with males of the same nonhuman species that have been administered with the compounds (or by whether pregnancy can be confirmed); whether fertilization is established when sperms collected from male nonhuman animals administered with the compounds are contacted with unfertilized eggs collected from females of the same animal species; or whether sperms collected from male nonhuman animals administered with the compounds fuse with naked hamster eggs.

The present invention also provides methods of screening for therapeutic agents for infertility, which use nonhuman animals in which the function of OBF gene is artificially suppressed. In such methods, test compounds are first administered to male nonhuman animals of the present invention. The administration of test compounds to male nonhuman animals of the present invention can be conducted by the methods described above. Then, whether the test compounds substitute for a function of OBF protein is assessed, and compounds that substitute for a function of OBF protein are selected by comparison with cases in which test compounds were not administered. Whether test compounds function instead of OBF protein can be assessed, for example, by examining the following: whether females of a nonhuman animal species have litters when mated with the above-described males of the same nonhuman species (or by whether pregnancy can be confirmed); whether fertilization is established when sperms collected from the above-described male nonhuman animals are contacted with unfertilized eggs collected from females of the same animal species; or whether sperms collected from the above-described genetically altered male nonhuman animals fuse with naked hamster eggs.

The present invention also provides nonhuman animals in which an OBF gene function is artificially suppressed. Such nonhuman animals can be used as nonhuman model animals for infertility in the above-described screenings for therapeutic agents for infertility. The present invention also provides cells of nonhuman animals in which an OBF gene function is artificially suppressed. Such cells include, for example, sperms and spermatids. In particular, the sperms of nonhuman animals in which an OBF gene function is artificially suppressed can serve as model sperms for infertility.

In the present invention, "OBF gene function is artificially suppressed" typically refers to a state in which OBF gene expression is suppressed due to gene mutations such as nucleotide insertions, deletions, and substitutions being comprised in either or both of the gene alleles; however, OBF gene function can also be artificially suppressed by methods using antisense DNA, methods using DNAs encoding ribozymes, methods using RNAi technology, and so on.

Cases where a mutant OBF protein is expressed whose function as a normal OBF protein is decreased or lost are also included in the "suppression of OBF gene function". "Suppression" includes cases where the expression of only one gene allele is suppressed, in addition to cases where OBF gene expression is completely suppressed. In the present invention, the sites of such a gene mutation are not particularly limited, as long as gene expression is suppressed, and such sites include, for example, exons and promoters.

In the present invention, species from which the animals that are subject of OBF gene alteration are derived are generally nonhuman mammals, preferably rodents, such as mice, rats and hamsters, and rabbits and pigs. Of these, mice are particularly preferable.

The means for artificially suppressing OBF function in the nonhuman animals of the present invention include, for example, methods for entirely or partially deleting an OBF gene, and methods for entirely or partially deleting the expression regulatory region of an OBF gene. In a preferable method, an OBF gene is inactivated by inserting foreign genes into either or both of the alleles of the OBF gene. Therefore, in a preferred embodiment of the present invention, the nonhuman animals comprise foreign genes inserted into either or both of the OBF gene alleles.

The nonhuman animals of the present invention can be generated by genetic engineering techniques commonly known to those skilled in the art. For example, mice in which a gene function is suppressed can be generated by the following procedure: first, DNAs comprising OBF gene exon portions are isolated from mice and appropriate marker genes are inserted into these DNA fragments to construct targeting vectors. These targeting vectors are introduced into mouse ES cell lines by electroporation or the like and cell lines in which homologous recombination has occurred are selected. Preferable marker genes for insertion are antibiotic resistance genes, such as neomycin resistance gene. When an antibiotic resistance gene is inserted, cell lines in which homologous recombination has occurred can be selected simply by culturing in a medium containing an antibiotic. Further, for more efficient selection, a thymidine kinase gene or the like can be linked with the targeting vectors. In this way, cell lines in which nonhomologous recombination has occurred can be removed. Alternatively, cell lines in which one of the OBF gene alleles is inactivated can be efficiently obtained by examining homologous recombinants using PCR and Southern blotting.

When selecting cell lines in which homologous recombination has occurred, there is a fear of unknown gene disruptions due to gene insertion at other sites than the site of homologous recombination. Therefore, chimeras are preferably generated using multiple clones. Chimeric mice can be obtained by injecting the obtained ES cell lines into mouse blastoderms. These chimeric mice can be mated to obtain mice in which one of the OBF gene alleles is inactivated. Furthermore, by mating these mice, mice in which both of the OBF gene alleles are inactivated can be obtained. By the same technique, genetic alteration can be carried out in animals other than mice for which ES cells have been established.

Furthermore, ES cell lines in which both OBF gene alleles have been inactivated can be obtained by the following method: specifically, by culturing ES cell lines in which one of the alleles is inactivated in a medium containing a high concentration of an antibiotic, cell lines in which the other allele is also inactivated, namely ES cell lines in which both OBF gene alleles are inactivated, can be obtained. Alternatively, they can also be generated by selecting ES cell lines in which one of the alleles is inactivated, introducing once again a targeting vector into the cell lines, and selecting cell lines in which homologous recombination has occurred. The marker genes to be inserted into the targeting vectors are preferably different from the marker genes described above. Sperms in which both OBF gene alleles have been inactivated can also be obtained by differentiating the ES cells generated.

The present invention also provides methods for testing infertility, where the methods comprise the step of measuring OBF gene expression level. Herein, "OBF gene expression" comprises not only the expression of OBF mRNAs, but also the expression of OBF proteins.

Examples of embodiments of the test methods of the present invention are described below; however, the test methods of the present invention are not limited thereto. In an embodiment of the above-mentioned test methods, first, RNA samples are prepared from subjects. The RNA samples can be extracted, for example, from the sperms or testes of subjects.

In the present methods, the amount of RNA encoding OBF protein comprised in the RNA sample is then measured. Then, the measured amount of RNA is compared with that of a control. Such methods include, for example, Northern blotting, DNA array, and RT-PCR.

The test methods described above can be conducted by measuring the OBF protein expression level, as in the following procedure: first, protein samples are prepared from subjects. Protein samples can be prepared, for example, from the sperms or testes of subjects.

In the present methods the amount of OBF protein comprised in the protein samples is then measured. Then, the measured amount of OBF protein is compared with that of the control. Examples of such methods include Western blotting using SDS-polyacrylamide gel electrophoresis and antibodies that bind to OBF proteins, dot blotting, immunoprecipitation, enzyme-linked immunosorbent assay (ELISA), and immunofluorescence.

The present invention also provides methods for testing infertility, which comprise the step of detecting mutations in an OBF gene region.

In the present invention, an OBF gene region refers to the region comprising an OBF gene and regions influencing expression of the gene. Regions influencing expression of the gene are not particularly limited, and include, for example, promoter regions.

Moreover, the type, number, site and so on of the mutations of the present invention is not limited, as long as the mutations cause a decrease or loss of sperm fertility. The types of the above-described mutations include, for example, deletions, substitutions, and insertion mutations.

Preferred embodiments of the test methods that comprise the step of detecting mutations that occurred in an OBF gene region are described below; however the methods of the present invention are not limited to these methods.

In a preferred embodiment of the test methods described above, first, DNA samples are prepared from subjects. DNA samples can be prepared, for example, based on RNAs or chromosomal DNAs.

In the present methods, DNAs comprising OBF gene regions are then isolated. The gene regions can be isolated, for example, by PCR or such using primers that hybridize to a DNA comprising the gene region, and using RNAs or chromosomal DNAs as templates.

In the present methods, the nucleotide sequences of the isolated DNAs are then determined. The nucleotide sequences of the isolated DNAs can be determined by methods known to those skilled in the art.

In the present methods, the determined DNA nucleotide sequences are then compared with a control. In the present invention, the control refers to a DNA comprising a normal (wild type) OBF gene region. In general, since the sequence of a DNA comprising an OBF gene region is assumed to be normal in healthy persons, the above "compared with a control" typically means a comparison with the sequence of a DNA comprising an OBF gene region of a healthy person.

The mutations of the present invention can also be detected by the following procedure: DNA samples are first prepared from subjects. Then, the prepared DNA samples are cleaved with restriction enzymes. Next, the DNA fragments are separated according to their size. Then, the sizes of the detected DNA fragments are compared with a control. Moreover, in an alternative embodiment, DNA samples are first prepared from subjects. Then, DNAs comprising an OBF gene region are amplified. Further, the amplified DNAs are cleaved with restriction enzymes. Then, the DNA fragments are separated according to their size, and the sizes of the detected DNA fragments are compared with a control.

Such methods include, for example, methods using restriction fragment length polymorphisms (RFLPs) and PCR-RFLP. Specifically, when there are mutations within a restriction enzyme recognition site or when nucleotide insertions or deletions exist in the DNA fragments that result from restriction enzyme treatment, the sizes of the fragments resulting after the restriction enzyme treatment change compared to a control. When a portion comprising this mutation is amplified by PCR and treated with each of the restriction enzymes, these mutations can be detected as a difference in band mobility after electrophoresis. Alternatively, the presence or absence of mutations can be detected by treating chromosomal DNAs with these restriction enzymes, electrophoresing, then carrying out Southern blotting using a probe DNA of the present invention. Restriction enzymes to be used can be appropriately selected according to each mutation. Apart from genomic DNAs, RNAs prepared from subjects can also be converted in this method into cDNAs using reverse transcriptase, and these can be directly cleaved with restriction enzymes to carry out Southern blotting. Moreover, DNAs comprising an OBF gene region can be amplified by PCR using these cDNAs as templates, these can be cleaved with restriction enzymes, then examined for differences in their mobility.

In an alternative method, DNA samples are first prepared from subjects. Then, DNAs comprising an OBF gene region are amplified. The amplified DNAs are then dissociated into single-stranded DNAs. Next, the dissociated single-stranded DNAs are separated on a non-denaturing gel. The mobilities on the gel of the fractionated single-stranded DNAs are compared a control.

Such methods include, for example, single-strand conformation polymorphism (PCR-SSCP) (Cloning and polymerase chain reaction-single-strand conformation polymorphism analysis of anonymous Alu repeats on chromosome 11. Genomics. 1992 Jan 1; 12(1): 139-146; Detection of p53 gene mutations in human brain tumors by single-strand conformation polymorphism analysis of polymerase chain reaction products. Oncogene. 1991 Aug 1; 6(8): 1313-1318; Multiple fluorescence-based PCR-SSCP analysis with postlabeling. PCR Methods Appl. 1995 Apr 1; 4(5): 275-282). The procedures of these methods are relatively simple and further, they use only small amounts of test compounds. Due to these and other advantages, the methods are particularly suited to screening many DNA samples. The principle is as follows: when a double-stranded DNA fragment is dissociated into single strands, each strand forms a specific conformation depending on its nucleotide sequence. When the dissociated DNA strands are electrophoresed in a polyacrylamide gel containing no denaturant, complementary single-stranded DNAs of the same length migrate to different positions depending on differences in their conformations. Even a single nucleotide substitution alters the conformation of a single-stranded DNA, thereby altering its mobility in polyacrylamide gel electrophoresis. Thus, the presence of mutations due to a point mutation, deletion, insertion, or such in a DNA fragment can be detected by detecting these differences in mobility.

Specifically, DNAs comprising an OBF region are first amplified by PCR or such. In general, portions to be amplified are preferably about 200-400 bp long. Those skilled in the art can carry out PCR after appropriately selecting the reaction conditions and such. In PCR, the amplified DNA products can be labeled using primers labeled with isotopes such as ³²P, fluorescent dyes, biotin, or such. Alternatively, the amplified DNA fragments can be labeled by carrying out PCR after adding, in the PCR reaction solution, substrate nucleotides labeled with isotopes such as ³²P, fluorescent dyes, biotin, or such. Furthermore, the amplified DNA fragments can also be labeled by adding substrate nucleotides labeled with isotopes such as ³²P, fluorescent dyes, biotin, or such using Klenow enzyme and such after the PCR reaction. The labeled DNA fragments obtained in this way are denatured by heating or such, and electrophoresed in a polyacrylamide gel that does not comprise denaturants such as urea. The conditions for DNA fragment separation can be improved by adding an adequate amount of glycerol (about 5-10%) to the polyacrylamide gel at this point. The conditions of electrophoresis change depending on the nature of each DNA fragment; however, the electrophoresis is generally carried out at room temperature (20-25°C), and if a preferable separation cannot be obtained, a temperature between 4-30°C and giving an optimal mobility is investigated. After electrophoresis, mobility of the DNA fragments is detected by autoradiography using X-ray films, by fluorescence-detecting scanners, or such, and analyzed. When bands of different mobility are detected, the bands are excised directly from the gel, re-amplified by PCR, then directly sequenced to confirm the presence of mutations. Even when not using labeled DNAs, the bands can be detected by staining the gel after electrophoresis using ethidium bromide, silver staining, or such.

In yet another method, DNA samples are first prepared from subjects. Then, DNAs comprising an OBF gene region are amplified. Further, the amplified DNAs are separated in a gel in which the concentration of a DNA denaturant is gradually increased. Next, the mobilities on the gel of the fractionated DNAs are compared with a control.

Such methods include, for example, the denaturing gradient gel electrophoresis (DGGE) method. In the DGGE method, a mixture of DNA fragments is electrophoresed in a polyacrylamide gel with a denaturant concentration gradient, and DNA fragments are separated based on differences in their instability. When an unstable DNA fragment with a mismatch migrates to a part in the gel with a certain concentration of denaturant, the DNA sequence around the mismatch partially dissociates into single strands due to its instability. Since the mobility of this partially dissociated DNA fragment becomes very low, a difference arises with the mobility of a perfectly double-stranded DNA which has no dissociated portion, and thus the two can be separated. Specifically, a DNA comprising an OBF gene region is amplified by PCR or such using the primers of the present invention or such, this is then electrophoresed in a polyacrylamide gel in which the concentration of a denaturant, such as urea, is gradually increased as the DNA migrates, and compared with a control. DNA fragments in which mutations exist will form single strands at positions with lower concentrations of denaturant, and since their mobility rate becomes extremely slow, the presence or absence of mutations can be detected by detecting these differences in mobility.

Yet another method first provides DNAs comprising an OBF gene region prepared from subjects, and a substrate on to which is immobilized a nucleotide probe that hybridizes to the DNAs.

In the present invention, the "substrate" refers to a board-shaped material onto which nucleotide probes can be immobilized. In the present invention, nucleotides comprise oligonucleotides and polynucleotides. The substrates of the present invention are not particularly limited, as long as nucleotide probes can be immobilized; however, substrates generally used in DNA array technologies can be preferably used. In general, DNA arrays are constituted by thousands of nucleotides printed at high density on to a substrate. Such DNAs are generally printed on the surface layer of non-porous substrates. In general, the surface layer of a non-porous substrate is made of glass; however, it is also possible to use porous membranes, for example, nitrocellulose membranes.

In the present invention, methods for immobilizing nucleotides (arrays) include, for example, arrays based on the oligonucleotide developed by Affymetrix. The oligonucleotides for the oligonucleotide arrays are generally synthesized *in situ.* Methods for synthesizing oligonucleotides *in situ* are already known and include, for example, ink jet technology (Rosetta Inpharmatics) for immobilizing chemical substances and photolithographic technology (Affymetrix). Any methods may be used to produce the substrates of the present invention.

The nucleotide probes to be immobilized onto substrates are not particularly limited, as long as they allow detection of mutations in an OBF gene region. Therefore, the probes are, for example, those that hybridize to DNAs comprising an OBF gene region. The nucleotide probes are not necessarily perfectly complementary to the DNAs comprising the gene regions, as long as they can specifically hybridize to them. In the present invention, when oligonucleotides are being immobilized, the nucleotide probes that are made to bind to the substrates are generally 10-100 bp long, preferably 10-50 bp long, and more preferably 15-25 bp long.

In the present methods, DNAs comprising a OBF gene region are then contacted with the substrate. With this process, the above-described nucleotide probes are hybridized to the DNAs. The reaction solution and reaction conditions for hybridization may vary depending on various factors, such as the length of the nucleotide probes to be immobilized onto the substrate; however, methods generally known to those skilled in the art can be used.

In the present methods, the intensity of hybridization between the DNAs comprising an OBF gene region and the nucleotide probes immobilized on to the substrate is detected next. This detection can be carried out, for example, by reading fluorescent signals using a scanner or such. In DNA arrays, the DNAs immobilized on to glass slides are generally called "probes", while the labeled DNAs in a solution are called "targets". Therefore, herein, the above-described nucleotides immobilized onto a substrate are referred to as "nucleotide probes". In the present methods, the detected intensity of hybridization is further compared with a control.

Such methods include, for example, DNA arrays (Strategies for SNP genetic mutations, Matsubara, K. and Sakaki, Y., Nakayama Shoten, p. 128-135; Nature Genetics (1999) 22:164-167).

In addition to the methods described above, allele specific oligonucleotide (ASO) hybridization can be used to detect just the mutations at specific sites. An oligonucleotide comprising a nucleotide sequence assumed to contain a mutation is produced, and hybridized to the DNAs. If mutations exist, the efficiency of hybrid formation is reduced. These can be detected by Southern blotting, methods using the characteristic of quenching due to intercalation of a special fluorescent reagent into gaps in the hybrid, and such.

In addition, the following methods can also be used in the present invention: MALDI-TOF/MS (Strategies for SNP genetic polymorphisms, Matsubara, K. and Sakaki, Y, Nakayama Shoten, p. 106-117; Trends Biotechnol (2000) 18:77-84), TaqMan PCR (Strategies for SNP genetic polymorphisms, Matsubara, K. and Sakaki, Y., Nakayama Shoten, p. 94-105; Genet Anal. (1999) 14:143-149), invader assays (Strategies for SNP gene polymorphisms, Matsubara, K. and Sakaki, Y, Nakayama Shoten, p. 94-105; Genome Research (2000) 10:330-343), pyrosequencing (Anal. Biochem. (2000) 10:103-110), AcycloPrime methods (Genome Research (1999) 9:492-498), single nucleotide primer extension (SNuPE) methods (Rapid Vommun Mass Spectrom. (2000) 14:950-959), and such.

Furthermore, the present invention provides test agents for use in the test methods of the present invention. In an embodiment, the test agents comprise oligonucleotides with a length of at least 15 nucleotides and which hybridize to an OBF gene region.

The oligonucleotides hybridize specifically to DNAs (normal or mutant DNAs) comprising OBF gene regions. Herein, "hybridize specifically" means that there is no significant cross-hybridization to DNAs encoding other proteins under standard hybridization conditions, preferably under stringent hybridization conditions (for example, the conditions described in Sambrook J. et al., "Molecular Cloning", 2nd Ed., Cold Spring Harbor Laboratory Press, New York, USA, 1989). Perfect complementarity is not necessary, as long as the oligonucleotides can hybridize specifically to a DNA comprising an OBF gene region.

Oligonucleotides with a length of at least 15 nucleotides and which hybridize to DNAs comprising an OBF gene region can be used as probes (comprising substrates onto which probes have been immobilized) or primers in the above-described test methods of the present invention. When the oligonucleotides are used as primers, they are typically 15-100 bp long, and preferably 17-30bp long. The primers are not particularly limited, as long as they allow amplification of at least a portion of an OBF gene region comprising a mutation site.

When the above-described oligonucleotides are used as probes, the probes are not particularly limited as long as they hybridize specifically to DNAs comprising an OBF gene region. The probes may be synthetic oligonucleotides, and typically have a length of at least 15 nucleotides.

The oligonucleotides of the present invention can be produce, for example, using commercially available oligonucleotide synthesizers. Probes may also be produced as double-stranded DNA fragments obtained by restriction enzyme treatment or such.

The oligonucleotides of the present invention are preferably used as probes after appropriately labeling. Labeling methods include, for example, labeling methods in which the 5' end of an oligonucleotide is phosphorylated with ³²P using T4 polynucleotide kinase, and methods that comprise using a DNA polymerase, such as Klenow enzyme to incorporate substrate nucleotides labeled with isotopes such as ³²P, fluorescent dyes, biotin, or the like, using a random hexamer oligonucleotide or such as primers (random priming methods and the like).

In an alternative embodiment of the present invention, the test agents comprise antibodies that recognize an OBF antigen of the present invention. The above antibodies are not particularly limited, as long as they are antibodies that can be used in tests. The antibodies are labeled, as required.

In addition to oligonucleotides and antibodies that are active ingredients, for example, sterile water, physiological saline, vegetable oils, detergents, lipids, solubilizers, buffers, protein stabilizers (BSA, gelatin, and such), and preservatives may be mixed in the above-described test agents, as required.

All prior art references cited herein are incorporated by reference into this description.

### Examples

Herein below, the present invention will be specifically described with reference to Examples, but it is not to be construed as being limited thereto.

### [Example 1] Experimental materials and methods

### 1. Production of anti-sperm antibody and analysis of in vitro fertilization inhibition using the antibody

### 1-1. Production of OBF13 monoclonal antibody

Sperm from the epididymis tails of male C57BL/6 mice 12 weeks old or older (Japan SLC) were suspended in PBS(-) and washed twice with PBS(-). Freund's complete adjuvant and Freund's incomplete adjuvant (Difco) were each mixed with the sperm (1 x 10⁶) and respectively injected at the start of immunization and 21 days later into the peritoneal cavities of female C57BL/6 mice for immunization. The third immunization (day 28) was carried out using a mixture of sperm and PBS(-). On day 32, spleen cells (1 x 10⁸) were extracted from mouse spleens, and fused with 1 x 10⁷ cells of the myeloma cell line P3U1 using polyethylene glycol 4000 (Sigma Chem.). Hybridomas were selected using selection medium comprising Dulbecco's Modification of Eagle's Medium (DMEM) (Flow Lab) supplemented with 100 IU/ml penicillin, 100 µg/ml streptomycin (Flow Lab), 1 µg/ml Fungizone (Flow Lab), 80 µg/ml Gentacin (Shionogi Pharm. Co., Ltd.), 2 x 10⁻⁵ M 2-mercaptoethanol (Nakalai Chem.), 15% fetal calf serum (M.A. Bioproducts), and HAT (hypoxanthine, aminopterin and thymidine). Clones producing sperm-specific antibodies were selected by fluorescent staining of the sperms using culture supernatants and 96-well plates.

Of these, hybridomas (1 x 10⁸ cells) producing an antibody (OBF13) strongly reactive to sperms were injected into the peritoneal cavities of CBF1 mice, which had been administered with 0.5 ml of pristane (Sigma Chem.) 14 days earlier, and then ten to 14 days later ascites fluids were collected (Okabe, M., et al. J Reprod Immunol 11, 91-100 (1987)).

### 1-2. Fluorescent staining of sperms

Mouse sperms from the epididymis were cultured for two hours in mouse sperm culture medium (TYH) (Toyoda, Y, et al. Jpn J Anim Reprod 16, 147-51 (1971)), then 1 x 10⁵ of the resulting sperms were reacted with 50 µl of culture supernatant obtained from the hybridomas (OBF13) for two hours at 37°C. After washing several times using PBS(-), FITC-conjugated anti-mouse IgM (Miles-Yeda Ltd.) was diluted to 25 µg/ml with a solution in which 5% NBCS (new-born calf serum) was added to PBS, and 20 µl of the resulting solution was reacted with the sperms for one hour. After washing several times with PBS(-), these sperms were observed under a fluorescent microscope.

### 1-3. Inhibition of in vitro fertilization using OBF13 monoclonal antibody

5 IU of pregnant mare serum gonadotropin (PMSG) (Teikoku Zoki) was injected into the peritoneal cavities of female BDF1 mice eight weeks old or older. After 48 hours, the mice were injected with 5 IU of human chorionic gonadotropin (hCG) (Teikoku Zoki) to encourage superovulation. Unfertilized eggs were collected from the ampulla of the fallopian tube after 13 to 15 hours. The unfertilized eggs were used directly, or after the cumulus cells were removed using 0.01% (w/v) hyaluronidase (Sigma Chem.). Sperms were extracted from the epididymis, and cultured in TYH medium at 37°C for one hour. Then, OBF13 monoclonal antibody was added to the sperms, incubated for one hour, and then added to the medium containing eggs at a final concentration of 2 x 10⁵ sperms/ml. Further, the fertilized eggs in TYH medium were distinguished using pronucleus formation as an indicator under a phase contrast microscope with the Hoffman modulation contrast.

### 2. OBF gene cloning

### 2-1. Two-dimensional electrophoresis

Epididymides and spermatic ducts were excised from male ICR mice twelve weeks old or older (ten animals). After collection, the sperms were solubilized using Lysis buffer (1% TritonX-114, PBS, 10 mM Benzamidine, 1 mM PMSF, 1 µg/ml Pepstatin, and 1 µg/ml Leupeptin). The insoluble fraction was removed by centrifugation, then the solubilized protein was overlaid onto 6% sucrose, and allowed to stand at 37°C for five minutes. After centrifugation at room temperature and 600 x g for 15 minutes, 2D buffer (8 M Urea, 2% CHAPS, and 1 mg/ml OrangeG) was added to the bottom layer fraction, suspended, centrifuged at 20,000 x g for 20 minutes, and then the supernatant was desalted using a desalting column. IPG buffer was added at 0.5% to 400 µl of the sample. Immobiline DryStrip pH4-7 (Amersham) was swollen in an Immobiline DryStrip Reswelling Tray for twelve hours. After isoelectric focusing electrophoresis using Multiphor II (Amersham), the DryStrip was soaked in Equilibration Buffer (50 mM Tris/HCl (pH6.8), 6M Urea, 30% glycerol, and 1% SDS) for 20 minutes, and then subjected to SDS-PAGE using 7.5% polyacrylamide gel under non-reducing conditions. After electrophoresis, the gel was silver-stained, and Western blotting was carried out using 1 µg/ml of OBF13 antibody.

### 2-2. Sequencing

A spot reactive to OBF13 identified in two-dimensional electrophoresis was stained with CYPRO Ruby protein gel stain (Molecular Probes, Inc.), and then excised from the gel. Peptide sequencing was carried out using LC-MS/MS. The mRNA sequence and amino acid sequence (accession number: XM_133424) of the antigen for OBF13 were determined based on the peptide sequence by analyzing sequences in NCBI database. Further, to confirm the accuracy of these sequences, RT-PCR was carried out using total RNAs extracted from mouse testes as a template, and the DNA was sequenced. In addition for human OBF, based on analysis of homology to mouse OBF, a putative gene for human OBF was identified (accession number: BC034769), and its sequence was determined by the same method as used for mice.

### 3. Generation of OBF polyclonal antibodies and in vitro functional analysis of OBF

### 3-1. Production of cell lines constitutively expressing human or mouse OBF

OBF antigens were produced to generate polyclonal antibodies against human and mouse OBF. To express the OBF proteins, plasmid DNAs were produced by constructing DNA sequences having a Kozak sequence at the N-terminus and a histidine tag at the C-terminus which were then inserted into the mammalian expression vector pCXN2 (Niwa, H., et al. Gene 108, 193-9 (1991)). These plasmid DNAs were introduced into cells of the rabbit kidney cell line RK13 (RIKEN Cell Bank) using lipofectamine 2000 (Invitrogen). Cells selected using 600 µg/ml G418 (Invitrogen) were then cloned using penicillin cups. The cells were maintained using Dulbecco's Modification of Eagle's Medium (DMEM) (Invitrogen) with 100 IU/ml penicillin, 100 µg/ml streptomycin, 2 mM L-glutamine, 0.1 mM MEM non-essential amino acids (Invitrogen), and 10% fetal calf serum (JRH Biosciences).

### 3-2. Production of human and mouse OBF polyclonal antibodies

OBF proteins as antigens were produced using the cell lines generated as described in 3-1. 1 x 10⁷ cells were collected from these cell lines using 10 mM EDTA, washed several times with PBS(-), and suspended in 0.5 ml of PBS(-). 0.6 ml of Freund's complete adjuvant (Difco) was added to this PBS(-) suspension, an emulsion was formed, and rabbits were immunized at the lymph nodes of both legs once a week, for a total of four times. Boosting was carried out after the fourth immunization, and blood was collected from the rabbits. Serum IgG was precipitated using ammonium sulfate at a final concentration of 33%, dialyzed against PBS(-), and then stored at -80°C (Inoue, N., et al. J Immunol 166, 424-31 (2001)).

### 3-3. Western blotting using various mouse tissues

Proteins of various mouse tissues were solubilized by suspending each tissue at 100 mg/ml in lysis buffer (1% (v/v) Triton X-100, PBS, 10 mM Benzamidine, 1 mM PMSF, 1 ug/ml Pepstatin, and 1 µg/ml Leupeptin), then crushing using a Potter-type homogenizer. The solubilized samples were centrifuged at 4°C and 20,000 x g for 30 minutes. The soluble fractions were collected, and proteins in the fractions were quantified by the CBB method using equivalent amount of BAS. 30 µg was fractionated by SDS-PAGE (10% polyacrylamide gel) and then transferred onto a polyvinylidene difluoride (PVDF) membrane. After transfer, the membrane was blocked with blocking buffer (0.15% Tween 20, 0.5 M NaCl, and 20 mM Tris-HCl (pH 7.4)) containing 10% skimmed milk. Then, 1 µg/ml of mouse OBF polyclonal antibody was added to the blocking buffer, and reacted at room temperature for two hours. After washing several times with membrane washing solution (0.1% (v/v) Tween 20, 0.5 M NaCl, and 20 mM Tris-HCl (pH 7.4)), the membrane was reacted in blocking buffer containing peroxidase-labeled anti-rabbit IgG (at a 10,000-times dilution; Jackson ImmunoResearch) at room temperature for one hour. After the membrane was washed again with membrane washing solution, color development and detection was carried out using the ECL system (Amersham).

### 3-4. Analysis of fusion between mouse sperms and eggs using antibodies

Unfertilized eggs were prepared by the same procedure as described in 1-3, and the zona pellucida was removed using a piezomanipulator (Yamagata, K., et al. Dev Biol 250, 348-57 (2002)). The naked eggs were incubated for ten minutes in a paraffin oil-covered TYH drop (100 µl) containing Hoechst 33342 at a final concentration of 1 µg/ml. The naked eggs were transferred into another drop, and allowed to stand at 37°C for 30 minutes to remove excess Hoechst 33342 (Molecular probes). This process was repeated three times. Sperms from epididymides were cultured in TYH for two or six hours, and then added to the Hoechst 33342-treated naked eggs at a final concentration of 2 x 10⁵ sperms/ml. Further, one hour before insemination 25 µg/ml OBF 13 monoclonal antibody, 25 µg/ml OBF polyclonal antibody, or 25 µg/ml CD9 polyclonal antibody (KMC8.8, Santa Cruz Biotechnology, Inc.) was added to the drops containing the sperms and eggs. When sperms fuse with eggs, Hoechst 33342 transfers from the eggs to the heads of the sperms, which become fluorescent. These sperms were assumed to be fused sperms, and thus counted using a fluorescent microscope.

### 4. Generation and analysis of OBF-knockout mice

### 4-1. Generation of OBF-knockout mice

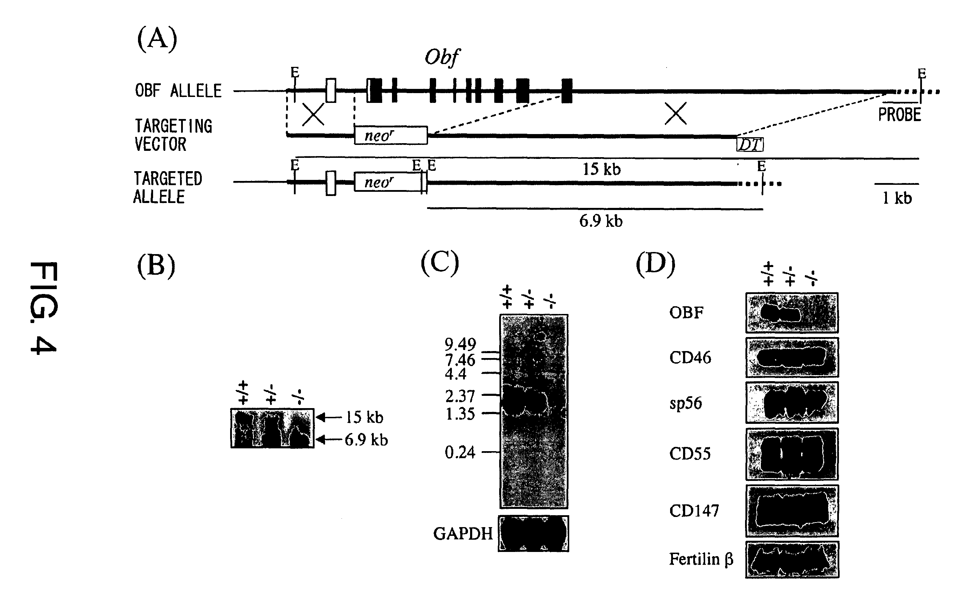

To inactivate the function of OBF, a targeting vector was produced comprising the neomycin resistance gene substituting the region of exon 2 to 10, as shown in Fig. 4A, and this was introduced into D3 embryonic stem (ES) cells by electroporation. After selection with 150 µg/ml G418 (Invitrogen), four positive colonies were selected by PCR using the genomic DNA. Then, four clones of the cell lines in which homologous recombination had occurred as expected were selected by Southern blotting. The four types of cell lines were injected into C57BL/6 mouse embryos at the blastocyst stage. As a result, chimeric mice able to transmit the targeting allele to their progenies were obtained. OBF heterozygote mice (F1 mice) were obtained by mating the mice with wild type mice. Mating between F1 mice then gave OBF-knockout mice (F2 mice). Subsequent analyses were carried out mainly using F2 mice, or F3 mice obtained by mating between F2 mice.

### 4-2. Southern blotting

Genomic DNAs were extracted from ES cells and mouse tails, and then digested with EcoRI. After the DNAs were electrophoresed in 0.8% agarose gel using 1x TAE buffer (0.04 M Tris, 0.04 M CH₃COOH, and 1 mM EDTA (pH 8.0)), the gel was denatured using denaturation buffer (0.5 N NaOH, and 1.5 M NaCl), and then neutralized using neutralization buffer (0.5 M Tris-HCl (pH 7.5), and 3M NaCl). The DNAs were transferred onto a nylon membrane (Hybond-N+; Amersham). The transfer membrane was incubated at 60°C for one hour in hybridization buffer (Express Hybridization buffer, Clontech) containing DNA labeled with ³²P, where the DNA was constructed on the outer side of the long arm. The transfer membrane was washed several times with membrane washing solution (washing solution 1: 2x SSC and 0.05% SDS; washing solution 2: 0.1x SSC and 0.1% SDS), and then was autoradiographed onto X-ray films (Hyper film MP, Amersham).

### 4-3. Northern blotting

Total RNAs were extracted from testes using TRIZOL Reagent (Invitrogen). 20 µg of each was separated in 1% agarose gel containing 1x MOPS and 18% formaldehyde. The gel was denatured using a denaturation buffer (50 mM NaOH), followed by neutralization using neutralization buffer (200 mM CH₃COONa). The samples were transferred onto a nylon membrane (Hybond-N+, Amersham). The RNA-transferred nylon membrane was incubated with a ³²P-labeled full-length ORF OBF fragment in hybridization buffer (Express Hybridization buffer, Clontech) at 68°C for one hour. The transfer membrane was washed several times with membrane washing solutions (washing solution 1: 2x SSC, 0.05% SDS; washing solution 2: 0.1x SSC,0.1% SDS), and then was autoradiographed onto X-ray films(Hyper film MP, Amersham).

### 4-4. Western blotting

The western blotting procedure was the same as described in 3-3. Testes and sperms were collected from 12-week-old mice of each genotype, and proteins were solubilized using lysis buffer. 30 µg of each protein was separated by SDS-PAGE using 10% acrylamide. Each protein was detected using 1 µg/ml OBF polyclonal antibody, 10 µg/ml mouse CD46 polyclonal antibody (Inoue, N., et al. Mol Cell Biol 23, 2614-22 (2003)), 0.1 µg/ml sp56 monoclonal antibody (QED Bioscience Inc.), 1 µg/ml mouse CD55 polyclonal antibody (gifted by Professor Hidechika Okada of Nagoya university), 0.4 µg/ml CD147 polyclonal antibody (Santa Cruz Biotechnology, Inc.), or 1 µg/ml Fertilin β monoclonal antibody (9D, CHEMICON International).

### 4-5. Average number of littermates

12-week-old or older male OBF +/- and -/- mice and 8-week-old or older female C57BL6 or OBF-/-mice were placed in the same cages. After confirmation of plugs, the average number of littermates was determined based on the number of littermates delivered naturally. Eight or more pairs were used for each genotype, and two or more births were observed per pair.

### 4-6. In vitro fertilization and analysis of fusion between sperms and eggs

*In vitro* fertilization was carried out using the same method as described in 1-3; however, unfertilized eggs bound with cumulus cells were used without removal of cumulus cells using hyaluronidase. Fusion between sperms and eggs was analyzed by the same method as in 3-4.

### 4-7. Analysis of fusion using naked hamster eggs

Seminal fluid was manually collected from healthy male volunteers. The seminal fluid was liquefied by incubation at 37°C for one hour. 0.5 ml each of liquefied seminal fluid was placed at the bottom of a test tube containing 2 ml of BWW medium (Overstreet, J.W., et al. Fertil Steril 33, 534-42 (1980)). Test tubes were incubated for one hour at an angle of 30°. Sperm swimming up were collected from 1.5 ml of the supernatant, and washed once with BWW. The sperms were suspended in 200 µl of BWW. The suspension was added to a 200 µl drop (400 µl in total), and the sperms were incubated at 37°C for six hours. During the incubation, frozen hamster eggs (Nosan Co.) were thawed and the zona pellucida was removed using a piezomanipulator (Yamagata, K., et al. Dev Biol 250, 348-57 (2002)). OBF+/- or OBF-/sperms, or sperms treated with 25 µg/ml anti-human OBF polyclonal antibody were incubated at 37°C at a final concentration of 1 x 10⁶ sperms/ml with naked hamster eggs. After six hours, swollen sperm heads were stained with 1 µg/ml Hoechst 33342, and fused sperms were counted and observed under a fluorescent microscope.

### 4-8. Intracytoplasmic sperm injection (ICSI)

Eggs were prepared by the same method as used in the *in vitro* fertilization, and cumulus cells were removed using 0.01% (w/v) hyaluronidase (Sigma Chem.). The unfertilized eggs were washed several times with drops of kSOM (Ho, Y., et al. Mol Reprod Dev 41, 232-8 (1995) ), then transferred into another kSOM drop and stored in an incubator at 37°C under 5% CO₂. Sperms were collected from epididymis tails of OBF +/- and -/- mice, and cultured in TYH at 37°C for one hour. Then, sperms at a concentration of 1 x 10⁷ sperms/ml were aliquoted in 10 µl quantities into tubes and frozen in liquid nitrogen. In ICSI, 12% (w/v) PVP/H-CZB (Chatot, C.L., et al. J Reprod Fertil 86, 679-88 (1989)), containing about 2 µl of thawed sperms, was added in the form of spots to the cover of a 6 cm dish. Several spots of FHM (Lawitts, J.A., & Biggers, J.D. J Reprod Fertil 91, 543-56 (1991)) were placed on the same dish, and the unfertilized eggs were placed into them. Sperm heads alone were introduced into the cytoplasm of the unfertilized eggs with an injection needle of about 7 µm in diameter, using a piezomanipulator (Kimura, Y. & Yanagimachi, R. Biol Reprod 52, 709-20 (1995)). The unfertilized eggs were cultured in kSOM for 24 hours, then transferred into the oviducts of pseudopregnant female mice on day 0.5 of the two-cell stage. After 19 days, newborn mice were obtained by caesarean section or natural delivery.

### 5. Generation and analysis of OBF-rescue mice

### 5-1. Generation of OBF-rescue mice

To confirm that the cause of the male sterility of OBF-knockout mice is the deletion of OBF protein, transgenic mice expressing OBF in their testes were generated and by mating female OBF-/- mice with the transgenic mice, male OBF -/- mice with an OBF transgene were generated. The vector used for the mouse OBF-transgenic mouse was produced to allow the expression under the regulation of the calmegin promoter, which is expressed in a testis-specific manner (Ikawa, M., et al. Dev Biol 240, 254-61 (2001)). The transgenic mice were generated by either of the following methods: the fertilized eggs were obtained by mating male OBF+/-mice and female OBF-/- mice after superovulation treatment, and the vector was injected into the pronuclei of the fertilized eggs at the pronucleus stage; or, alternatively, the gene was introduced into the fertilized eggs at the two-cell stage using lentivirus infection (Ikawa, M., et al. Mol Ther 8, 666-73 (2003)).

### 5-2. Measurement of the fertilization rate of eggs in the oviduct

Superovulated mice were prepared by the method as described in 1-3. After injection of hCG, the mice were placed in the same cages with male mice for mating. The male mice used were: (1) OBF +/-; (2) OBF -/-; and (3) double-transgenic OBF-/- mice with the OBF transgene. The following morning, the vaginas of the females were examined. After confirming plugs, which show mating behavior, eggs were collected from the ampulla of the fallopian tube. Eggs with abnormal cytoplasm were also removed at this time. The fertilized eggs were distinguished using pronucleus formation as an indicator under a phase contrast microscope using Hoffman modulation contrast.

### [Example 2]

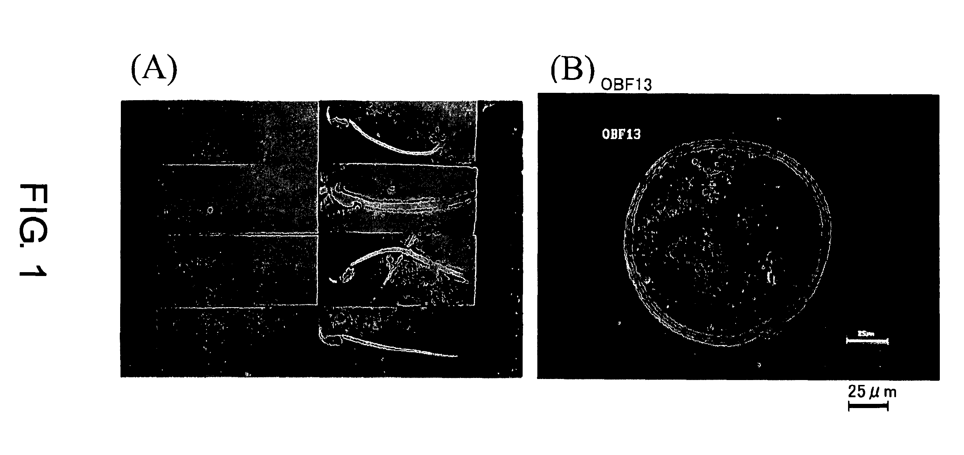

The present inventors extracted sperms from the epididymides of male C57BL/6 mice, immunized female mice of the same line using these sperms, and generated many monoclonal antibodies to exhaustively identify the sperm membrane proteins that contributed to the mechanism of fertilization. From these one clone was obtained, that of a unique monoclonal antibody that reacted specifically only to sperms after acrosome reaction, where the form is changed due to fusion of the sperm cell membrane with the acrosomal outer membrane to undergo capacitation. This antibody (OBF13) is reactive to an antigen that gradually spreads all over the sperm head as the acrosome reaction progresses (Fig. 1A). The antibody was reactive to the entire sperm head after penetration through the zona pellucida of egg (Fig. 1B). Furthermore, the treatment of sperms with OBF13 had influence on neither binding to the zona pellucida nor penetration through it; however, the treatment had an inhibitory effect on subsequent fusion with eggs in an antibody concentration-dependent manner (Tables 1 and 2).

**Table 1**

| Effect of OBF13 monoclonal antibody on the penetration of sperms through the zona pellucida in an in vitro fertilization system | | | |
|---|---|---|---|
| Dilution fold of ascites | Number of experiments | Total number of eggs used in experiments | Eggs having sperms in the perivitelline space^{a)}(%) |
| - | 9 | 209 | 65±7.7 |
| 1/10000 | 9 | 214 | 66±6.2 |
| 1/2000 | 9 | 211 | 51±7.2 |
| 1/400 | 9 | 201 | 51±9.5 |
| 1/80 | 9 | 220 | 55±6.7 |

| | | | |
|---|---|---|---|
| a) Numeric values are indicated as mean ± standard deviation | | | |

**Table 2**

| Effect of OBF13 monoclonal antibody on the fusion between sperms and eggs in an in vitro fertilization system | | | |
|---|---|---|---|
| Dilution fold of ascites | Number of experiments | Total number of eggs used in experiments | Eggs having both female and male pronuclei^{a)} (%) |
| - | 9 | 210 | 59±11.2 |
| 1/10000 | 9 | 223 | 17±3.8** |
| 1/2000 | 9 | 202 | 0 |
| 1/400 | 9 | 214 | 0 |
| 1/80 | 9 | 221 | 0 |

| | | | |
|---|---|---|---|
| a) Numeric values are indicated as mean ± standard deviation ** p<0.01 : Statistically significantly different to the control at a 1% or lower risk ratio. | | | |

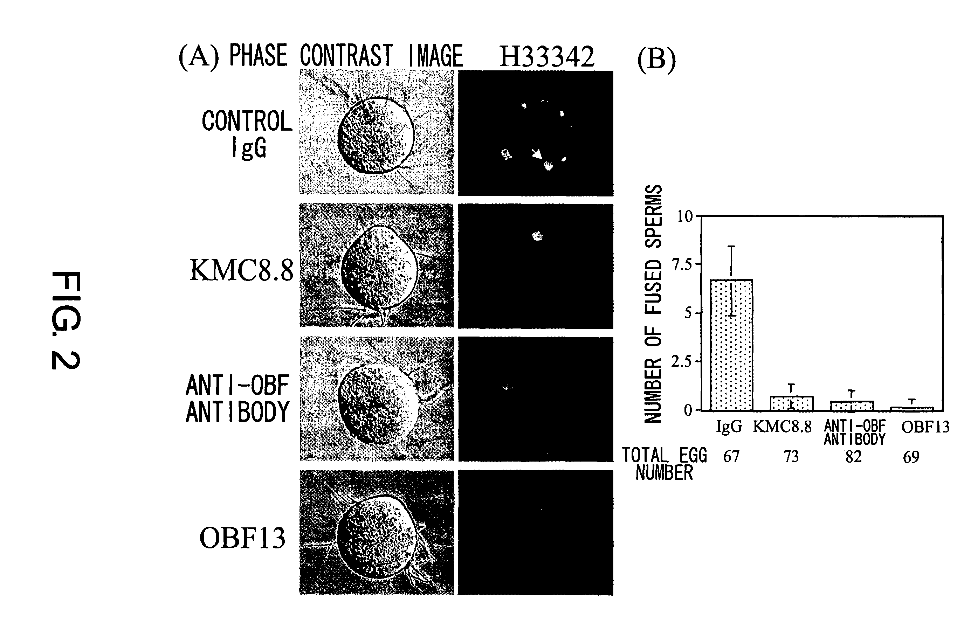

Specifically, the following was demonstrated: when Hoechst 33342 was incorporated into naked eggs and sperms were added to the eggs, Hoechst 33342 was found to transfer into sperms upon fusion with eggs; however, in fertilization inhibition experiments with OBF13 monoclonal antibody and mouse OBF polyclonal antibody, the number of fused sperms was significantly reduced as compared to the control IgG, although the binding of sperms to eggs was normal. This effect was the same as when anti-mouse CD9 antibody (KMC8.8) was added (Fig. 2).

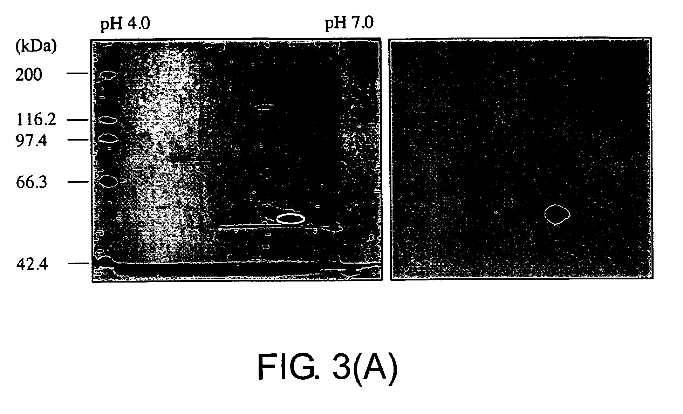

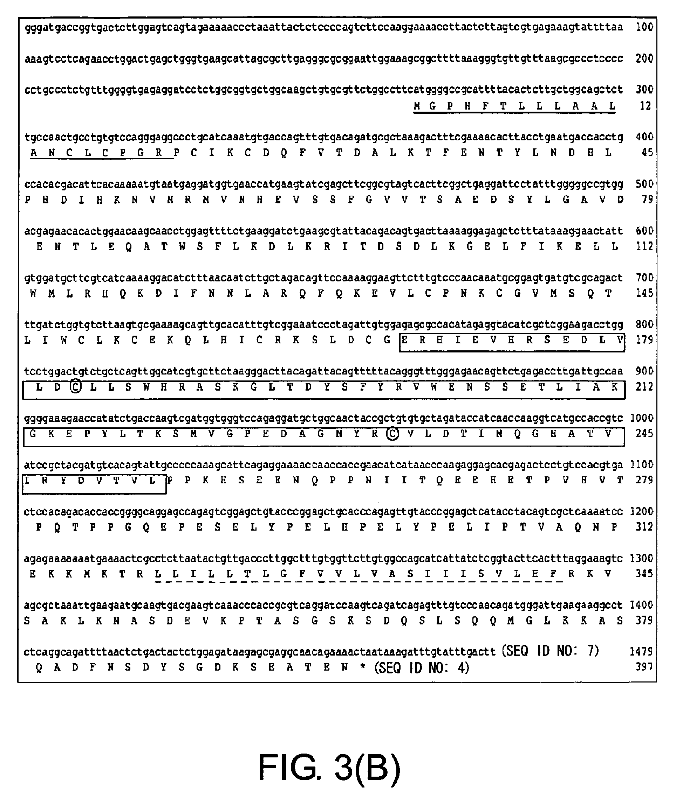

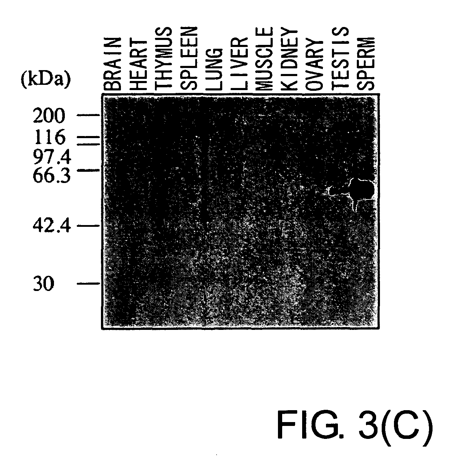

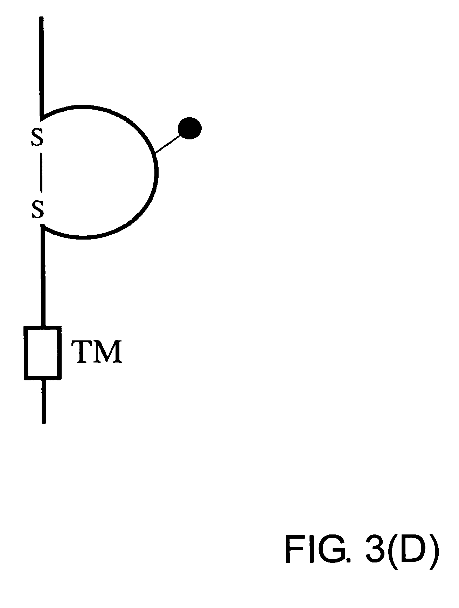

The present inventors assumed that the antigen recognized by the antibody is a molecule essential for the binding or fusion of sperms with eggs. To identify the antigen, the inventors collected sperms from the epididymides of ten male ICR mice, then solubilized the sperms using Triton X-114, a detergent allowing rough extraction into a membrane fraction and an aqueous fraction. Then, the membrane fraction was separated by two-dimensional electrophoresis (the first dimension: pH 4.0 to 7.0; the second dimension: 7.5% polyacrylamide gel for SDS-PAGE). The sample was transferred onto PVDF membrane, and the positions of antigen spots was identified by Western blotting using OBF13 (Fig. 3A). The identified spot was then excised from the gel, and the primary structure was determined by LC-MS/MS (Fig. 3B).

A cDNA (accession number: XM_133424) comprising the entire ORF for the OBF13 antigen had been deposited as a molecule with unknown function in the NCBI database. Therefore, the mRNA sequence could be deduced from it. To confirm the accuracy of this, the sequence was determined by RT-PCR and DNA sequencing using total RNA extracted from mouse testes as a template. Motif analysis of this molecule (OBF (oocyte binding/fusion factor)) revealed it was a typical type-I membrane protein comprising a signal sequence, extracellular domain, transmembrane domain, and cytoplasmic domain (Fig. 3B). Furthermore, the results of a database search revealed that OBF existed in not only mice, but also at least humans and rats, and they shared a high homology of more than 57% at the amino acid level. The nucleotide sequences of human, mouse, and rat OBF are shown in SEQ ID NOs: 1, 3, and 5, and their amino acid sequences are shown in ID NOs: 2, 4, and 6. In all the animal species, the extracellular domain of OBF was found to have a single Ig-like (immunoglobulin-like) domain, and thus the domain was assumed to be formed via disulfide bonds between cysteines (Fig. 3D). In addition, the N-linked glycosylation site in this domain was completely conserved in all of the animal species.

According to the EST database, mouse OBF has been shown to be expressed in the testes and brain; however, a polyclonal antibody against mouse OBF was produced and each tissue was tested using western blotting, which revealed that OBF was present specifically in the testis and sperms at a protein level (Fig. 3C).

Knockout mice were generated and analyzed to demonstrate that OBF indeed serves as a molecule involved in the fusion *in vivo.* Knockout mice were generated by constructing a targeting vector to substitute the neomycin resistance gene for exon 2 to 10 of the OBF gene, which consists of 10 exons, and then cloning ES cells in which homologous recombination has occurred by using standard methods (Fig. 4A). The presence of homologous recombination in each mouse was tested by Southern blotting, northern blotting, and western blotting (Fig. 4B to 4D). The results demonstrated that mice in which homologous recombination had occurred lack of mRNA and protein derived from the OBF. As far as it was tested, the lack of OBF protein did not result in any secondary effects, such as the lack of other sperm membrane proteins (CD46: acrosomal inner membrane protein, sp56: acrosomal matrix protein, CD55; cell membrane protein, CD147; Ig-superfamily protein, fertilin β; protein involved in binding to the zona pellucida), or the reduction of expression levels thereof (Fig. 4D).

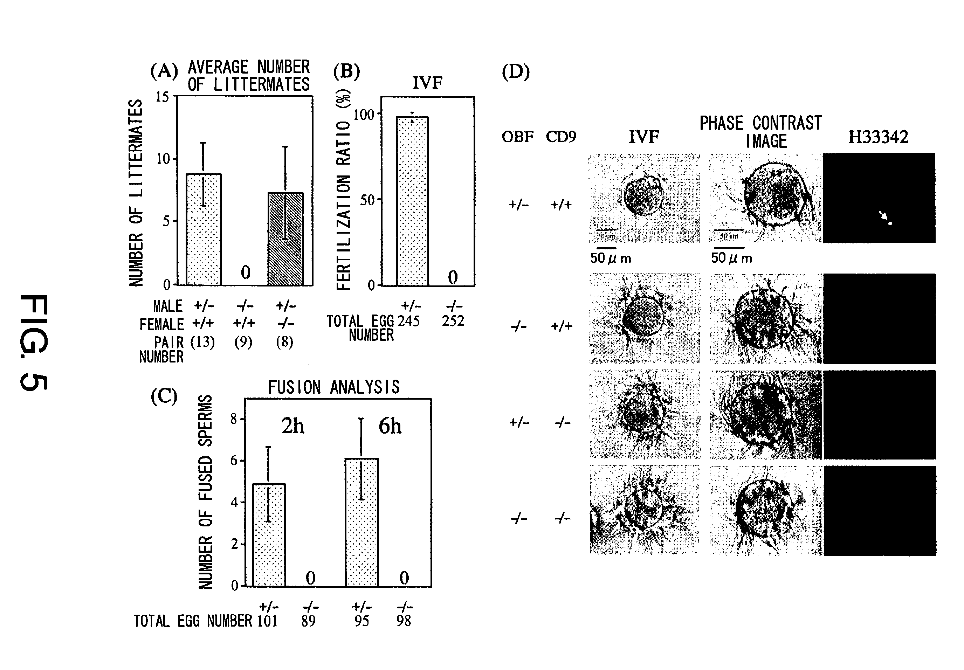

To test the fertility of male OBF-/- mice, the number of littermates was determined after natural mating with female C57BL/6 mice. As a result, a normal number of littermates was obtained from female OBF-/- mice, but no mice were obtained from male OBF-/- mice; thus, male OBF-/- mice were found to be infertile (Fig. 5A). To identify the cause of infertility in the male OBF-/- mice, detailed study was carried out using an *in vitro* fertilization system. As a result, sperms of male OBF-/- mice were confirmed to diffuse cumulus cells, and to bind to and penetrate the zona pellucida; however, no post-fertilization two-cell embryo was obtained (Fig. 5B). In addition, a study using naked eggs showed that OBF-/- sperms could bind to eggs but had no fusion ability (Fig. 5C). Further, even when OBF-/- sperms were added to CD9-/- eggs that were incapable of fusing, the sperms could bind to the eggs; thus, this suggests that binding and fusion are not concurrent phenomena, but can be discriminated as different events (Fig. 5D). One cause of infertility in male mice knocked-out for calmegin, fertilin, or ACE is known to be the inability of the sperms to swim up the oviduct. OBF-/- sperms were shown to swim up normally because sperms were found in an area surrounding the egg and perivitelline space of the ampulla of the fallopian tube after mating with female mice. Further, testes size and number of sperms, shape, and motility were all normal.

If the cause of infertility in male OBF-/- mice is limited to the lack of fusion ability and if the fusion process can be bypassed, subsequent fertilization should occur normally and litters should be obtained. To confirm this, OBF-/- sperms were subjected to ICSI (intracytoplasmic sperm injection). As a result, healthy litters were obtained form OBF-/- sperms (Table 3).

**Table 3**

| | Number of eggs subjected to ICSI | Number of viable eggs after injection | Number of eggs developed up to the two-cell stage | Number of littermates |
|---|---|---|---|---|
| OBF -/- | 95 | 59 | 42 (71%) | 12 (29%) |
| OBF +/- | 85 | 54 | 43 (80%) | 6 (14%) |

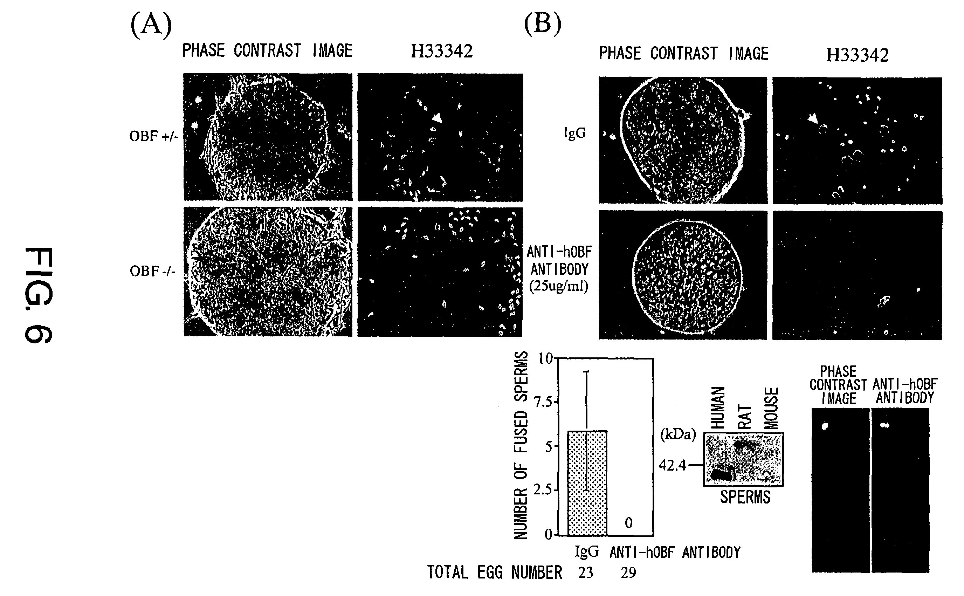

Sperms of various animal species are known to be capable of fusing with naked hamster eggs. Mouse OBF-/- sperms were used to test fusion with naked hamster eggs to investigate the variety of OBFs among animal species. The results showed that OBF-/- sperms could bind but could not fuse at all to the eggs, as was the case when using mouse eggs (Fig. 6A). Further, a human OBF polyclonal antibody was produced, and its influence on the fusion between human sperms and naked hamster eggs was tested. The results showed that human sperms could not even bind to naked hamster eggs, let alone fuse with them (Fig. 6B). These results demonstrate that OBF is a molecule essential for binding and fusion to eggs in fertilization across animal species.

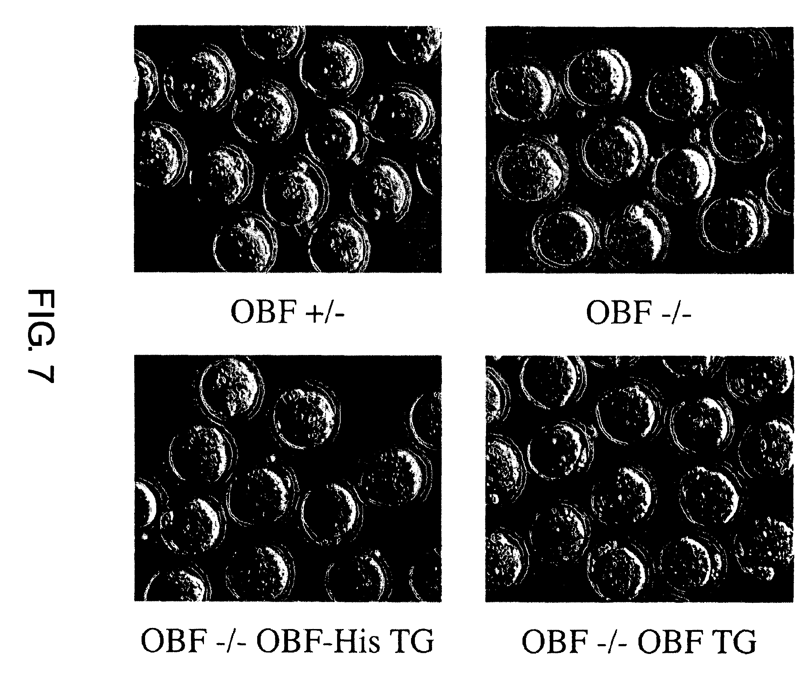

Finally, OBF-knockout mice were rescued using transgenic mice introduced with a vector expressing OBF under the regulation of the promoter of calmegin, a testis-specific molecular chaperon. Fertility was confirmed to be recovered, and the phenotype of infertility observed in OBF-/- mice was confirmed to be due to the deletion of the OBF gene (Fig. 7).

The above findings revealed that OBF is an important molecule crucial to binding and fusion to eggs, which are the most critical steps of fertilization, not only in mice but also in humans. Furthermore, since the antibody has the activity of inhibiting fertilization, OBF can be used as an antigen for contraceptive vaccines. Alternatively, OBF is also applicable for infertility diagnosis by testing OBF.

### Industrial Applicability

The present invention provides OBF proteins that are essential for binding or fusion between sperms and eggs, as well as antibodies against the proteins, and uses thereof. OBFs can be used as contraceptive vaccines, while antibodies against OBFs can be used as contraceptives. The present invention can also provide methods of screening for contraceptives and testing for infertility by using as an indicator the expression of an OBF protein or such. Further, nonhuman animals in which OBF gene functions are suppressed can be used as animal models for infertility.

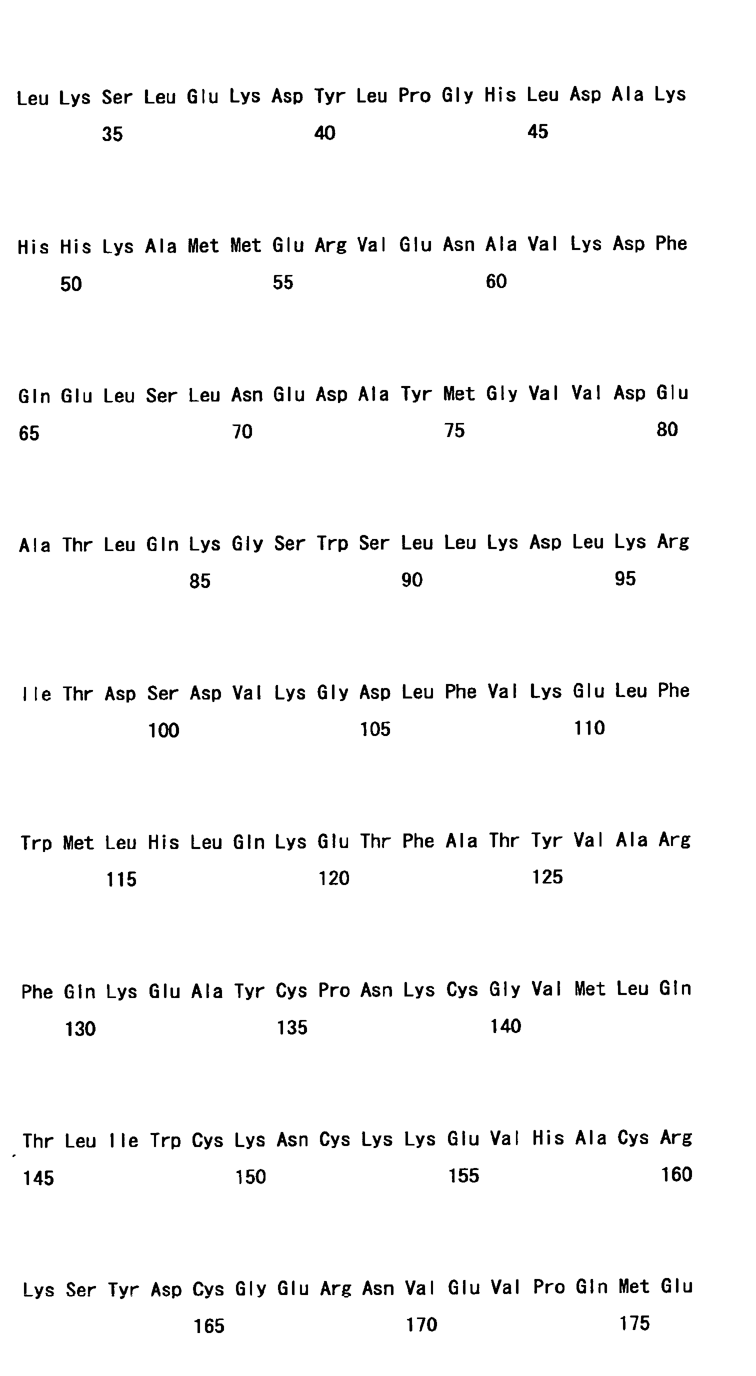

## Claims

1. An antibody (other than an OBF13 antibody) with the activity of inhibiting binding or fusion between a sperm and an egg, whose antigen is the protein described in any one of (a) to (d) below, or a fragment thereof:
(a) a protein encoded by a DNA comprising the nucleotide sequence of any one of SEQ ID NOs: 1,3, and 5;
(b) a protein comprising the amino acid sequence of any one of SEQ ID NOs: 2, 4, and 6;
(c) a protein comprising an amino acid sequence with a substitution, deletion, insertion, and/or addition of one or more amino acids in the amino acid sequence of any one of SEQ ID NOs: 2, 4, and 6, which is functionally equivalent to a protein comprising the amino acid sequence of any one of SEQ ID NOs: 2, 4, and 6; and
(d) a protein encoded by a naturally derived DNA that hybridizes under stringent conditions to a DNA comprising the nucleotide sequence of any one of SEQ ID NOs: 1, 3, and 5, which is functionally equivalent to a protein comprising the amino acid sequence of any one of SEQ ID NOs: 2, 4, and 6.

2. A contraceptive comprising as an active ingredient an antibody with the activity of inhibiting binding or fusion between a sperm and an egg, whose antigen is the protein of any one of (a) to (d) below, or a fragment thereof:
(a) a protein encoded by a DNA comprising the nucleotide sequence of any one of SEQ ID NOs: 1, 3, and 5;
(b) a protein comprising the amino acid sequence of any one of SEQ ID NOs: 2, 4, and 6;
(c) a protein comprising an amino acid sequence with a substitution, deletion, insertion, and/or addition of one or more amino acids in the amino acid sequence of any one of SEQ ID NOs: 2, 4, and 6, which is functionally equivalent to a protein comprising the amino acid sequence of any one of SEQ ID NOs: 2, 4, and 6; and
(d) a protein encoded by a naturally derived DNA that hybridizes under stringent conditions to a DNA comprising the nucleotide sequence of any one of SEQ ID NOs: 1, 3, and 5, which is functionally equivalent to a protein comprising the amino acid sequence of any one of SEQ ID NOs: 2, 4, and 6.

3. A contraceptive vaccine comprising as an active ingredient the protein of claim 2 or a fragment thereof.

4. A use of the protein of claim 2, or a fragment thereof, as a factor involved in binding or fusion between a sperm and an egg.

5. A method of screening for a candidate compound for a contraceptive, which comprises the steps of:
(a) contacting a test compound with the protein of claim 2 or a fragment thereof;
(b) detecting binding between the test compound and the protein of claim 2 or a fragment thereof; and
(c) selecting a test compound that binds to the protein of claim 2 or a fragment thereof.

6. A method of screening for a candidate compound for a contraceptive, which comprises the steps of:
(a) contacting a test compound with a cell comprising a DNA encoding the protein of claim 2;
(b) measuring the expression level of the DNA encoding the protein of claim 2 in the cell; and
(c) selecting a compound that reduces the expression level of the DNA as compared to when the test compound is not contacted.

7. A method of screening for a candidate compound for a contraceptive, which comprises the steps of:
(a) administering a test compound to a nonhuman animal comprising a DNA encoding the protein of claim 2;
(b) measuring the expression level of the DNA encoding the protein of claim 2 in the nonhuman animal; and
(c) selecting a compound that reduces the expression level of the DNA as compared to when the test compound is not administered.

8. A method of screening for a candidate compound for a contraceptive, which comprises the steps of:
(a) providing a cell or a cell extract comprising a DNA in which a reporter gene is operatively linked downstream of a promoter region of a gene encoding the protein of claim 2;
(b) contacting a test compound with the cell or cell extract;
(c) measuring the expression level of the reporter gene in the cell or cell extract; and
(d) selecting a compound that reduces the expression level of the reporter gene as compared to when the test compound is not contacted.

9. A method of screening for a candidate compound for a contraceptive, which comprises the steps of:
(a) contacting a sperm of a male animal with a compound selected by using a combination of one or more of the methods of claims 5 to 8;
(b) confirming the fertility of the sperm; and
(c) selecting a compound that inhibits the fertility of the sperm.

10. A method of screening for a candidate compound for a contraceptive, which comprises the steps of:
(a) administering a nonhuman male animal with a compound selected by using a combination of one or more of the methods of claims 5 to 8;
(b) confirming the fertility of a sperm of the nonhuman male animal; and
(c) selecting a compound that inhibits the fertility of the sperm of the nonhuman male animal.

11. A nonhuman animal in which a function of a gene encoding the protein of claim 2 is artificially suppressed.

12. A nonhuman animal cell in which a function of a gene encoding the protein of claim 2 is artificially suppressed.

13. A method of screening for a candidate compound for treating infertility, which comprises the steps of:
(a) administering a test compound to the nonhuman animal of claim 11;
(b) assessing whether the test compound substitutes for a function of the protein of claim 2; and
(c) selecting a compound that substitutes for a function of the protein as compared to when the test compound is not administered.

14. A method of testing for infertility, which comprises the step of measuring the expression level of a gene encoding the protein of claim 2.

15. A method of testing for infertility, which comprises the step of detecting a mutation in a region of a gene encoding the protein of claim 2.

16. A test agent used in the method of claim 14 or 15, which comprises an oligonucleotide with a length of at least 15 nucleotides that hybridizes to a region of a gene encoding the protein of claim 2.

17. A test agent used in the method of claim 14, which comprises an antibody whose antigen is the protein of claim 2 or a fragment thereof.
